(19) Europäisches Patentamt
European Patent Office
Office européen des brevets

(11) Publication number: 0 007 294
A2

(12) **EUROPEAN PATENT APPLICATION**

(21) Application number: 79850061.7

(22) Date of filing: 26.06.79

(51) Int. Cl.³: C 07 C 93/06
A 61 K 31/135, A 61 K 31/275
C 07 C 93/14, C 07 C 97/10
C 07 C 121/75, C 07 C 121/78

(30) Priority: 30.06.78 SE 7807408

(43) Date of publication of application: 23.01.80 Bulletin 80/2

(84) Designated Contracting States: AT BE CH DE FR GB IT LU NL SE

(71) Applicant: Aktiebolaget Hässle
Fack
S-431 20 Mölndal 1(SE)

(72) Inventor: Byrnes, Eugene William
191 Nola Drive
Holden, Massachusetts 01520(US)

(72) Inventor: Samuelsson, Gustav Benny Roger
Tubavägen 1 B
S-435 00 Mölnlycke(SE)

(72) Inventor: Aberg, Axel Karl Gunnar
12 Sherburne Road
Vestboro, Massachusetts 01581(US)

(74) Representative: Wurm, Bengt Runio et al,
Patent and Trade Mark Department Ab Astra
S-151 85 Södertälje(SE)

(54) **Membrane stabilizing compounds having beta-receptor blocking activity, their preparation, method for treatment of arrhythmic conditions, and pharmaceutical preparations containing same.**

(57) The present invention relates to new potent membrane stabilizing compounds of the formula

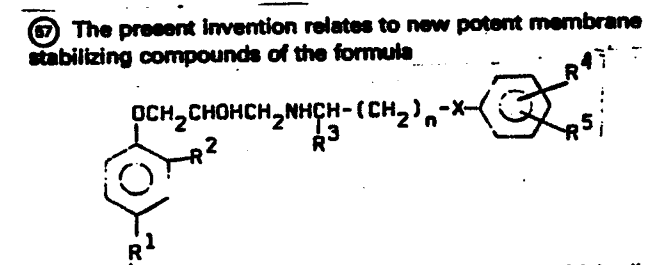

wherein $R^1$ is alkoxyalkyl, hydroxyalkyl or alkyl, which alkyl groups may be branched or straight, $R^2$ is halogen or hydrogen, $R^3$ is hydrogen or alkyl, $R^4$ and $R^5$ are same or different and are each hydrogen, alkoxy, alkyl, alkoxyalkyl, cyano, or hydroxy, n is 0, 1, 2, or 3, and X is -O-, $-CH_2-$, or $-\overset{O}{\overset{\|}{C}}-$.

The present compounds are potent heart antiarrhythmic agents possessing membrane stabilizing effect and β-receptor blocking effect in same clinical doses. The compounds are used in treating antiarrhythmic conditions, but can also be used in the treatment of hypertension, angina pectoris, or nervous heart. The invention also relates to processes for preparing the compounds, method of treating antiarrhythmic conditions and pharmaceutical preparations containing said compounds.

EP 0 007 294 A2

0007294

Aktiebolaget Hässle
Mölndal/SWEDEN

Inventors: E W Byrnes, G B R Samuelsson, G Åberg

KH 569-1
79-05-22
UI/EMH

# Membrane Stabilizing Compounds having β-receptor Blocking Activity, Their Preparation, Method for Treatment of Arrhythmic Conditions, and Pharmaceutical Preparations Containing Same

The present invention relates to new potent membrane stabilizing compounds as well as their preparation and a method for treating symptoms and signs of arrhythmic conditions by stabilizing membranes of the heart, hypertension, angina pectoris, nervous heart, by administering to mammals, including man, these new compounds.

The new compounds are those of the general formula

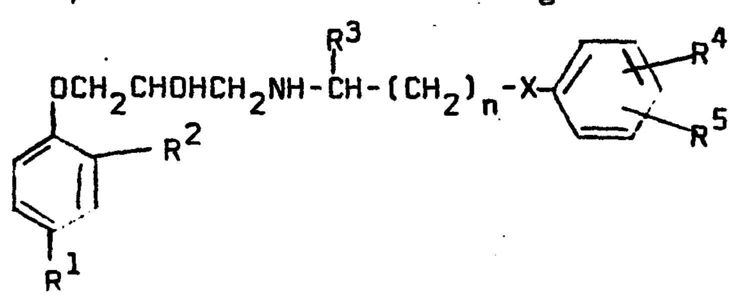

I

wherein $R^1$ is selected from the group consisting of alkoxyalkyl, hydroxyalkyl and alkyl, which alkyl groups may be branched or straight, $R^2$ is selected from the group consisting of halogen and hydrogen, $R^3$ is selected from the group consisting of hydrogen and alkyl, $R^4$ and $R^5$ are the same or different and are each selected from the group consisting of hydrogen, alkoxy, alkyl, alkoxyalkyl, cyano, and hydroxy, n is an integer 0, 1, 2, or 3, and X is -O-, $-CH_2$, or $-\overset{O}{\overset{\|}{C}}-$, whereby n is not zero when X is oxygen.

The alkoxyalkyl $R^1$ has up to 4 carbon atoms in each alkyl part, whereby each alkyl part may be branched or straight, and is, e.g., methoxymethyl, 2-methoxyethyl, 1-methoxyethyl, 3-methoxypropyl, 2-methoxypropyl, or 2-ethoxyethyl.

The hydroxyalkyl $R^1$ has up to 4 carbon atoms in the alkyl part, which alkyl part may be branched or straight, and is, e.g. hydroxymethyl, 2-hydroxyethyl, 3-hydroxypropyl, 1-hydroxyethyl, or 2-hydroxypropyl.

The alkyl $R^1$ has up to 4 carbon atoms and may be branched or straight, and is, e.g., methyl, ethyl, n-propyl, isopropyl, n-butyl, sec. butyl, isobutyl, or tert. butyl.

The halogen $R^2$ is chloro, bromo, iodo, or fluoro, preferably chloro, or bromo.

The alkyl $R^3$ has up to 4 carbon atoms and may be straight, or branched, preferably straight, and is methyl, ethyl, or propyl, preferably methyl.

The alkoxy $R^4$ and $R^5$ have up to 4 carbon atoms in the alkyl part thereof, and are, e.g., methoxy, ethoxy, isopropoxy, propoxy, or butoxy, preferably methoxy, and ethoxy.

The alkyl $R^4$ and $R^5$ have up to 4 carbon atoms and are, e.g., methyl, ethyl, or isopropyl.

The alkoxyalkyl $R^4$ and $R^5$ have up to 4 carbon atoms in each alkyl part, and are, e.g., methoxymethyl, methoxyethyl, ethoxyethyl, or isopropoxymethyl.

n is an integer 0, 1, 2, or 3, preferably 0 and 1, whereby n is not zero when X is oxygen.

X is oxygen, methylene, or carbonyl.

$R^4$ and $R^5$ are bound in any position on the phenyl group, whereby preferably $R^4$ is hydrogen and $R^5$ is p-hydroxy, and $R^4$ and $R^5$ are both hydrogen.

Several compounds with well known adrenergic β-receptor blocking activities, e.g., propranolol and alprenolol have membrane stabilizing properties as well. Since β-blockers and membrane stabilizers (local anesthetics) exert antiarryhthmic effect on the heart by different mechanisms (Vaughan-Williams, E.M. <u>in</u> Symposium on cardiac arrhythmias Astra 1970), they are effective against different types of arrhythmias. However, the β-blockers presently used in the clinic exert their β-blocking activity in blood plasma concentrations of 50-100 nanograms/ml while they exert their membrane stabilizing effect (local anesthetic activity) in blood plasma concentrations of several micrograms/ml.

The present invention relates to new compounds having valuable properties in affecting arrhythmic conditions of the heart by combining and at the same plasma concentration exerting both β-blocking and membrane stabilizing(local anesthetic) effects on the heart. Other indications calling for a treatment using the present compounds are hypertension, angina pectoris, nervous heart and other heart diseases.

Compounds according to the present invention are:
3-[2-phenethylamino]-1-[4-(2-methoxyethyl)phenoxy]-propanol-2;
3-[2-(4-hydroxyphenyl)-ethylamino]-1-[4-(2-methoxyethyl)-phenoxy]-propanol-2;

3-[2-phenethylamino]-1-[4-(2-methoxyethyl)-2-bromophenoxy]-propanol-2;

3-[1-methyl-2-(4-hydroxyphenyl)-ethylamino]-1-[4-(2-methoxyethyl)-2-bromophenoxy]-propanol-2;

3-[1-methyl-2-(4-hydroxyphenyl)-ethylamino]-1-[4-(2-methoxyethyl)-2-chlorophenoxy]-propanol-2;

3-[2-(4-hydroxyphenoxy)-ethylamino]-1-[4-(2-methoxyethyl)-phenoxy]-propanol-2;

3-[2-(4-hydroxyphenoxy)-ethylamino]-1-[4-(2-methoxyethyl)-2--bromophenoxy]-propanol-2;

3-[2-(4-hydroxyphenylcarbonyl)-ethylamino]-1-[4-(2-methoxyethyl)-phenoxy]-propanol-2;

3-[2-(3,5-dimethoxyphenyl)-ethylamino]-1-[4-(2-hydroxyethyl)-phenoxy]-propanol-2;

3-[2-(2-cyano-5-methylphenyl)-ethylamino]-1-(4-ethylphenoxy)-propanol-2;

3-[2-(3,5-diethylphenylcarbonyl)-ethylamino]-1-[4-ethyl-2-bromophenoxy]-propanol-2;

3-(2-phenethylamino)-1-[4-(2-methoxy-1-methylethyl)phenoxy]-propanol-2;

3-(2-phenethylamino)-1-[4-(methoxymethyl)phenoxy]-propanol-2;

or

3-(3-phenylpropylamino)-1-[4-(2-methoxyethyl)phenoxy]-propanol-2.

Salt forming acids may be used in preparing therapeutically acceptable salts of the compounds. These are: hydrohalogen acids, sulfuric acid, phosphoric acid, nitric acid, perchloric acid, aliphatic, alicyclic, aromatic or heterocyclic carboxylic or sulfonic acids, such as formic, acetic, propionic, succinic, glycolic, lactic, malic, tartaric, citric, ascorbic, maleic, hydroxymaleic, or pyruvic acid, phenylacetic, benzoic, p-aminobenzoic, anthranilic, p-hydroxybenzoic, salicylic or p-aminosalicylic acid, embonic acid, methanesulfonic, ethanesulfonic, hydroxyethane sulfonic, ethylenesulfonic, halogenbenzenesulfonic,

toluenesulfonic, naphtalenesulfonic, or sulfanilic acid, methionine, tryptophan, lysine or arginine.

The substances are intended to be administered orally or parenterally for acute and chronic treatment of the above mentioned cardiovascular disorder.

The biological effects of the new compounds have been tested, and the different tests carried out will be shown and explained below.

The new compounds are obtained according to methods known _per se_. Thus, a compound of formula II,

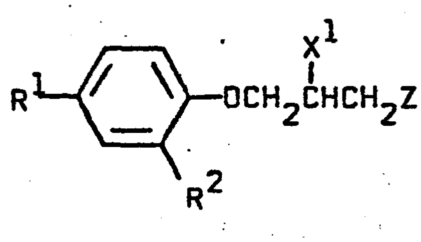

(II)

wherein $R^1$ and $R^2$ have the meanings given above, $X^1$ is a hydroxy group, Z is a reactive, esterified hydroxy group, or $X^1$ and Z together form an epoxy group, is allowed to react with an amine of the formula

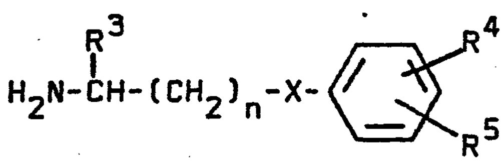

wherein $R^3$, $R^4$, $R^5$, n, and X have the same meanings as given above.

A reactive, esterified hydroxy group is particularly a hydroxy group esterified with a strong, inorganic or organic acid, preferably a hydrohalogen acid, such as hydrochloric acid,

hydrobromic acid, or hydroiodic acid, furthermore sulfuric acid or a strong organic sulfonic acid, e.g., benzenesulfonic acid, 4-bromobenzenesulfonic acid, or 4-toluenesulfonic acid. Thus, Z is preferably chloro, bromo or iodo.

This reaction is carried out in a known way. With the use of a reactive ester as a starting material, the preparation takes place preferably in the presence of a basic condensing agent and/or with an excess of an amine. Suitable basic condensing agents are, e.g., alkali metal hydroxides such as sodium or potassium hydroxide, alkali metal carbonates such as potassium carbonate, and alkali metal alcoholates such as sodium methylate, potassium ethylate, and potassium tert. butylate.

The reaction is carried out in an alkanol having 1 to 4 carbon atoms by refluxing the reactants in said solvent for a time long enough to produce the compound of formula I, generally 1 to 12 hrs.

Furthermore, a compound of formula III

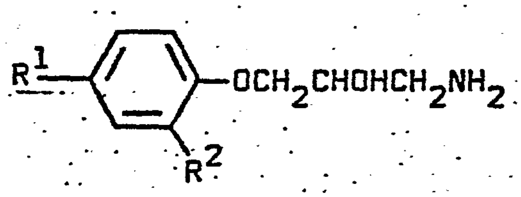

(III)

wherein $R^1$ and $R^2$ have the meanings given above, is allowed to react with a compound of the formula

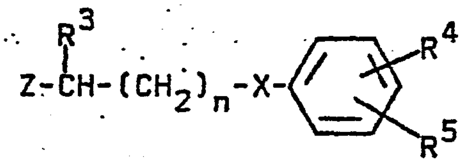

wherein $R^3$, $R^4$, $R^5$, n, X, and Z have the same meanings as given above.

This reaction is carried out in a known way, preferably in the presence of a basic condensing agent and/or an excess of an amine. Suitable basic condensing agents are, e.g., alkali metal alcoholates, preferably sodium or potassium alcoholate, or alkali metal carbonates such as sodium, or potassium carbonate.

This reaction is carried out in an alkanol having 1 to 3 carbon atoms in an autoclave heated to 100 to 130°C for 5 to 15 hrs.

Furthermore, a compound of formula IV

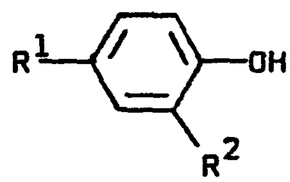

(IV)

wherein $R^1$ and $R^2$ have the same meanings as given above, is allowed to react with a compound of formula V

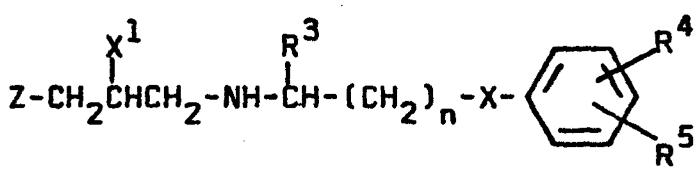

(V)

wherein Z, $X^1$, $R^3$, $R^4$, $R^5$, n, and X have the same meanings as given above.

This reaction is carried out in a known way. In those cases where reactive esters are used as starting material, the compound of formula IV may suitably be used in the form of its metal phenolate such as an alkali metal phenolate, preferably sodium phenolate, or by performing the reaction in the presence of an acid binding agent, preferably a condensing agent, which can form a salt of the compound of formula IV, such as an

alkali metal alcoholate.

This reaction is carried out in an alkanol having 1 to 3 carbon atoms in an autoclave heated to 80 to 100°C for 5 to 15 hrs.

Furthermore, a compound of formula Va

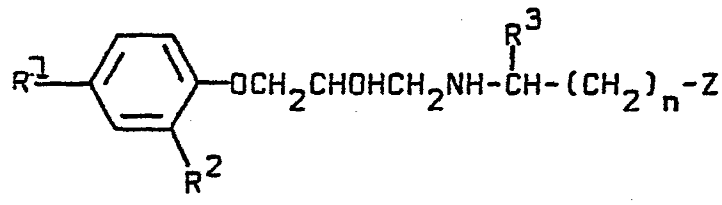

(Va)

wherein $R^1$, $R^2$, $R^3$, Z, and n have the meanings given above, is allowed to react with a compound of formula IVa

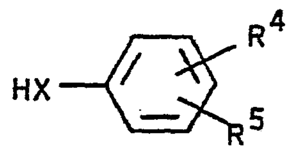

(IVa)

wherein $R^4$, and $R^5$ have the same meanings as given above, and X is O in the presence of a suitable base.

This reaction is carried out in the same way as the reaction between compounds of formula IV and V above.

Furthermore, a compound of formula IV

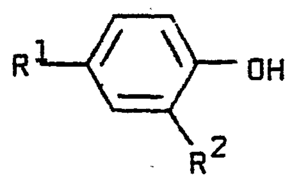

(IV)

wherein $R^1$ and $R^2$ have the same meanings as given above, is allowed to react with a compound of formula VI

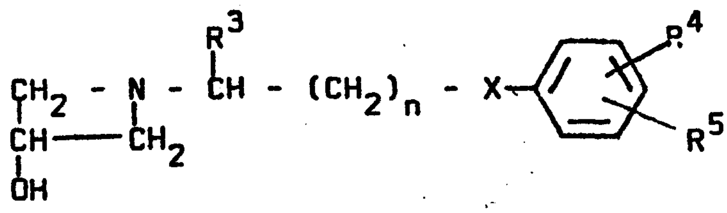

(VI)

wherein $R^3$, $R^4$, $R^5$, n and X have the same meanings as given above.

This reaction is carried out in a known way. Thus, the reaction is carried out under alkaline conditions in a suitable solvent, such as benzyl alcohol, by boiling the reaction mixture for some hours. Thereby the phenol is primarily converted to its metalphenolate such as alkali metal phenolate before it is added to the azetidinol of formula VI.

Furthermore, one may split off a residue from a compound of formula I above in which the nitrogen atom of the amino group and/or the hydroxy groups have attached thereto a removable residue.

Such removable residues are especially those which are removable by solvolysis, reduction, pyrolysis, or fermentation.

Residues removable by solvolysis are preferably residues removable by hydrolysis or ammonolysis.

Residues removable by means of hydrolysis are, e.g., acyl residues, which, when present, are functionally varied carboxyl groups, e.g., oxycarbonyl residues, such as alkoxycarbonyl residues, e.g., the tert. butoxycarbonyl residue, or the ethoxycarbonyl residue, aralkoxycarbonyl residues, such as phenylloweralkoxycarbonyl residues, e.g., a carbobenzyloxy residue, halogencarbonyl residue, e.g., a chlorocarbonyl residue; arylsulphonyl residues such as toluenesulphonyl or bromobenzenesulphonyl residues, possibly halogenated, such as fluorinated loweralkanoyl residues such as formyl-, acetyl-, or trifluoroacetyl residues, or a benzoyl residue or cyano groups or silyl residues such as trimethylsilyl residue.

Of the above mentioned residues present on the hydroxy groups, which residues are split off by hydrolysis, preferably the oxycarbonyl residues or the loweralkanoyl residues or the benzoyl residues are used.

Besides the above mentioned residues removable by hydrolysis also doubly bound residues, which are split off from the amino group by hydrolysis can be used, e.g., the alkylidene or benzylidene residue or a phosphorylidene group such as a triphenylphosphorylidene group, in which the nitrogen atom then obtains a positive charge.

Residues removable at the hydroxy group and the amino group by hydrolysis are furthermore divalent residues as in occurring cases substituted methylene. As substituents on the methylene residues any organic residue may be used, whereby it does not matter on hydrolysis which compound is the substituent to the methylene residue. As methylene substituents e.g. aliphatic or aromatic residues as alkyl as mentioned above, aryl, e.g., phenyl or pyridyl may be used. The hydrolysis may be carried out in any known way, suitably in a basic or preferably in an acid medium.

Compounds having residues being removable by hydrolysis are also the compounds according to formula VII.

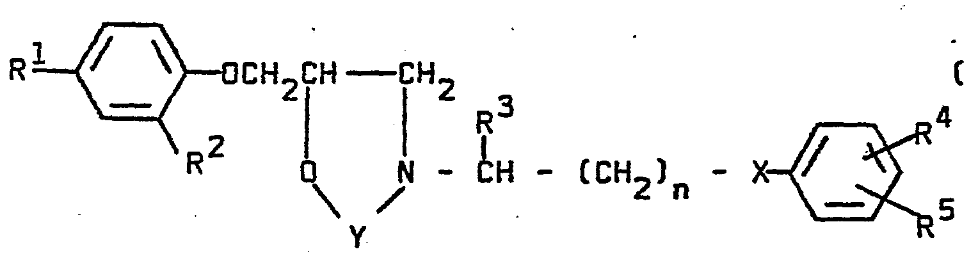

(VII)

wherein $R^1$, $R^2$, $R^3$, $R^4$, $R^5$, n, and X have the same meanings as given above and Y is a carbonyl or thiocarbonyl residue.

The hydrolysis is carried out in an analogous way, e.g., in the presence of a hydrolysing agent, e.g., in the presence of an acidic agent such as diluted mineral acids such as sulfuric

acid or hydrohalogen acid, or in the presence of basic agents such as e.g. alkali metal hydroxides, such as sodium hydroxide. Acidic agents are preferably used in the hydrolysis of compounds of formula VII.

Oxycarbonyl residues, aryl sulphonyl residues and cyano groups may in a suitable way be split off by means of acidic agents such as a hydrohalogen acid, preferably hydrobromic acid. Preferably the cleavage may take place using diluted hydrobromic acid, possibly in a mixture with acetic acid. Cyano groups are preferably split off by means of hydrobromic acid at an elevated temperature, such as in boiling hydrobromic acid, according to the "bromocyano method" (v. Braun). Furthermore, a tert. butoxycarbonyl residue, e.g., my be split off under anhydrous conditions by means of treatment with a suitable acid, such as trifluoroacetic acid.

Residues removable by ammonolysis are especially the halogencarbonyl residues, such as the chlorocarbonyl residue. The ammonolysis may be carried out in a known way, e.g. by means of an amine containing at least one hydrogen atom bound to the nitrogen atom, such as a mono- or diloweralkylamine, e.g., methylamine or dimethylamine, or especially ammonia, preferably at an elevated temperature. Instead of ammonia one may use an agent which gives ammonia such as hexamethylene tetraamine.

Residues removable by means of a reduction are, e.g., an α-arylalkyl residue, such as a benzyl residue or an α-aralkoxycarbonyl residue as a benzyloxycarbonyl residue, which in a known way may be split off by means of a hydrogenolysis, especially by catalytically activated hydrogen, such as by hydrogen in the presence of hydrogenating catalysts, e.g., Raney-nickel. Other residues removable by means of hydrogenolysis are 2-halogenalkoxycarbonyl residues, such as 2,2,2-trichloroethoxycarbonyl residue or 2-iodoethoxy- or 2,2,2-tribromoethoxycarbonyl residues, which may be split off in a known way, suitably by means of a metallic reduction (so called nascerating hydrogen). Nascerating hydrogen may be obtained by

the influence of metal or metal alloys, such as amalgam, on compounds which give hydrogen such as carboxylic acids, alcohols or water, whereby especially zinc or zinc alloys together with acetic acid may be used. Hydrogenolysis of 2-halogenalkoxycarbonyl residues may furthermore take place using chromium (II) compounds such as chromium (II) chloride or chromium (II) acetate.

A residue removable by reduction may also be an arylsulfonyl group such as a toluenesulfonyl group, which in a known way may be split off by reduction using nascerating hydrogen, e.g. by means of an alkalimetal, such as lithium or sodium in liquid ammonia, and suitably may be split off from a nitrogen atom. On carrying out the reduction one has to be careful that other reducible groups are not affected.

Residues removable by means of pyrolysis, especially residues removable from the nitrogen atom, are e.g., substituted, preferably unsubstituted, carbamoyl groups. Suitable substituents are e.g. loweralkyl or arylloweralkyl such as methyl or benzyl, or aryl, such as phenyl. The pyrolysis is carried out in a known way, whereby one must be careful that other thermally susceptible groups are not affected.

Residues removable by means of fermentation, especially residues removable from the nitrogen atom are e.g. substituted, however preferably unsubstituted, carbamoyl groups. Suitable substituents are, e.g., loweralkyl or arylloweralkyl, such as methyl or benzyl, or aryl, such as phenyl. The fermentation is carried out in a known way, e.g., by means of the enzyme urease or soy bean extract at about 20°C or slightly elevated temperature.

Furthermore, a Schiff's base of formula VIII or IX

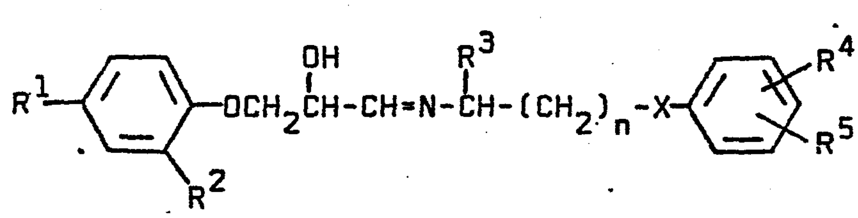

(VIII)

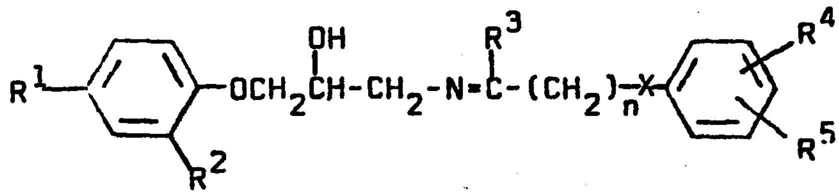

(IX)

or a cyclic tautomer X related to formula IX

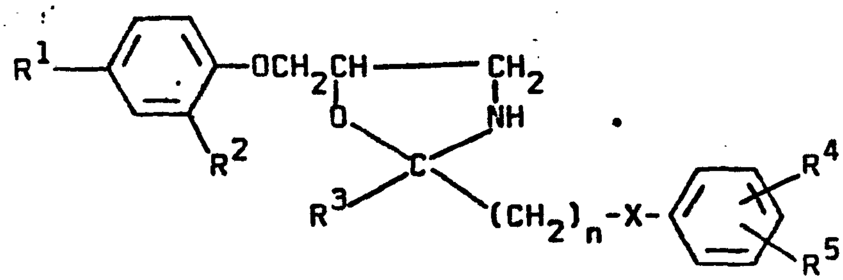

(X)

or a mixture of IX and X can be reduced, wherein $R^1$, $R^2$, $R^3$, $R^4$, $R^5$, n, and X have the same meanings as given above, except for the case when n = 0 and X = 0, too. This reduction is carried out in a known way, e.g., using a complex metal hydride, as sodium borohydride, lithiumaluminium hydride, using a hydride such as diborane with formic acid, or by means of catalytic hydrogenation, e.g., as with hydrogen in the presence of Raney-nickel. On reduction one has to be careful that other groups are not affected.

Further, the oxo group in the compound of formula XI

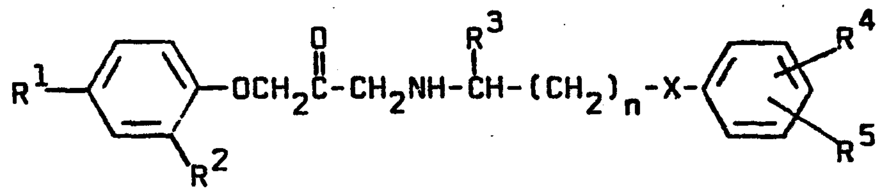

(XI)

wherein $R^1$, $R^2$, $R^3$, $R^4$, $R^5$, n, and X have the same meanings as given above, can be reduced to a hydroxy group. This reduction is carried out in a known way, especially using a complex metal hydride, as mentioned above, or according to the "Meerwein-Pondorf-Verley method" or a modification thereof, preferably using an alkanol such as isopropanol, as a reaction

component and as solvent, and using a metal alkanolate, such as metal isopropanolate, e.g. aluminium isopropanolate.

Furthermore, in a compound of formula XII

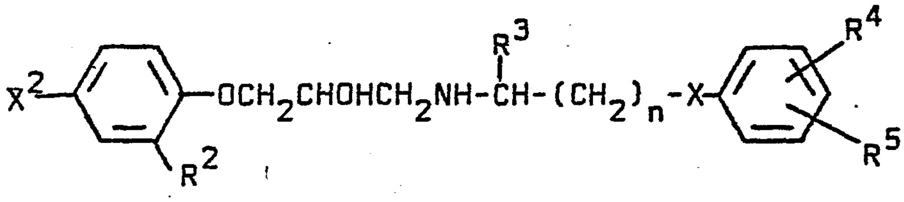

(XII)

wherein $R^2$, $R^3$, $R^4$, $R^5$, n, and X have the same meanings as given above, and wherein $X^2$ is a residue, which can be transformed into a residue $R^1$, one transforms $X^2$ into $R^1$, where $R^1$ has the same meaning as given above.

Thus, a ketal derivative of the formula XXII

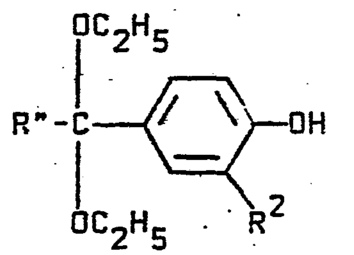

(XXII)

wherein $R^2$ has the meanings given above and R" is alkoxyalkyl, hydroxyalkyl or alkyl is reacted with epichlorohydrin, whereby the compound of formula XXIII

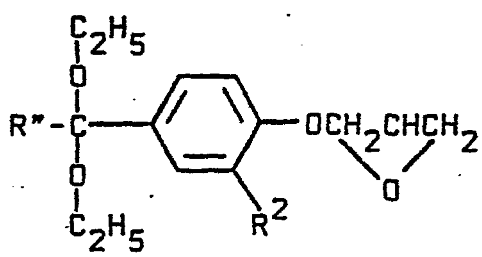

(XXIII)

wherein $R^2$, and R" have the meanings given above, is formed. The compound of formula XXIII is then reacted with a compound of the formula

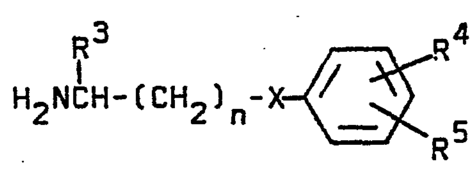

wherein $R^3$, $R^4$, $R^5$, X, and n have the meanings given above, whereupon the ketal is broken down in an acidic aqueous solution whereby a compound of the formula XXIV

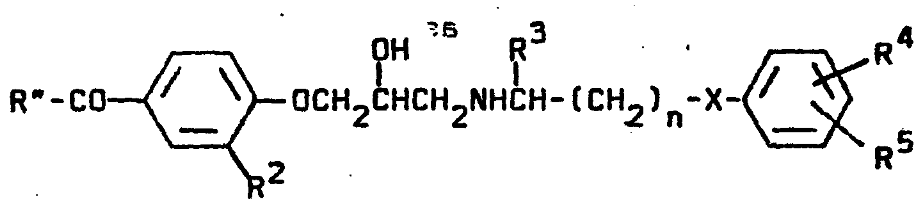

(XXIV)

wherein $R''$, $R^2$, $R^3$, $R^4$, $R^5$, X, and n have the meanings given above, is formed.

The compound of formula XXIV is then catalytically hydrogenated to give a compound of formula I. A suitable catalyst is palladium on active carbon, aluminium oxide, or barium sulphate. Solvents used in the hydrogenating step are lower alkanols, which may contain mineral acids, conc. acetic acid, or mixtures thereof. Depending on the solvent a suitable temperature between ambient temperature and 80°C and a suitable pressure between 1 and 5 atmospheres is chosen. The hydrogenation may be carried out only partly whereby the corresponding hydroxy compound is obtained. Other reducing methods are Clemensen and Wolff-Kishner reduction.

Thus $X^2$ may be a 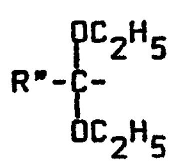 group,

or a $R''$-CO- group, or a 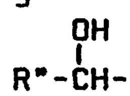 group.

$X^2$ may be a residue $HOR'''$ wherein $R'''$ is an alkylene group which can be etherified to give a compound of formula I wherein $R^1$ is alkoxy alkyl. A suitable reagent is then an alkyl halogenide or $X^2$ may be a residue $Z-R'''$, wherein Z and $R'''$ have the meanings given above, which is allowed to react with an alkanol to give a compound of formula I.

Furthermore, the oxo group in a compound XIII, related to formula I, which carries an oxo group at a carbon atom bound to a nitrogen atom, may be reduced to two hydrogen atoms.

Said compounds are, e.g., such ones as formula XIII

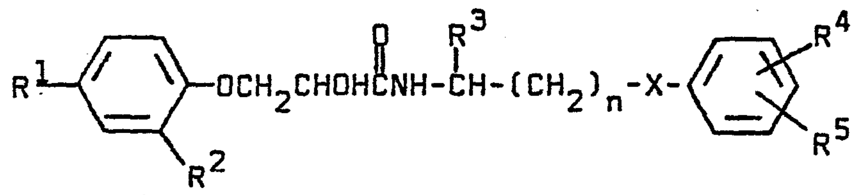

(XIII)

wherein $R^1$, $R^2$, $R^3$, $R^4$, $R^5$, n, and X have the meanings as given above.

The reduction can be carried out according to the above described manner using complex metalhydrides, e.g. lithium aluminium hydride or di-isobutylaluminium hydride. Suitably, the reaction may take place in an inert solvent such as an ether, e.g., diethylether or tetrahydrofuran.

Depending on the process conditions and the starting material the end product is obtained either in free form or in the form of its acid addition salt, which is included in the scope of the invention. Thus, for example, basic, neutral or mixed salts may be obtained as well as hemi, mono, sesqui, or polyhydrates. The acid addition salts of the new compounds may, in a manner known <u>per se</u>, be transformed into free compounds using, e.g., basic agents such as alkali or a suitable ion exchanger. On the other hand, the free bases obtained may form salts with organic or inorganic acids. In the preparation of acid addition salts, preferably such acids are used as form suitable therapeutically acceptable salts. Such acids are, e.g., hydrohalogen acids, sulfuric acid, phosphoric acid, nitric acid, perchloric acid, aliphatic, alicyclic, aromatic, or heterocyclic, carboxylic or sulfonic acids, such as formic, acetic, propionic, succinic, glycolic, lactic, malic, tartaric, citric, ascorbic, maleic, hydroxymaleic, or pyruvic acid, phenylacetic, benzoic, p-aminobenzoic, anthranilic,

p-hydroxybenzoic, salicylic or p-aminosalicylic acids; embonic acid, methanesulfonic, ethanesulfonic, hydroxyethanesulfonic, ethylenesulfonic acids; halogenbenzenesulfonic, toluenesulfonic, naphtalenesulphonic acids, or sulfanilic acid; methionine, tryptophane, lysine or arginine.

These or other salts of the new compounds, such as, e.g., picrates may serve as purifying agents, or the free bases obtained as the free bases, are transformed into salts, these are separated, and the bases are then set free from the salts again. According to the close relationship between the new compounds in free form and in the form of their salts it will be understood from the above and the below that, if possible, the corresponding salts are included when mentioning the free compound.

The invention also relates to any embodiment of the process in which one starts from any compound obtained as an intermediate in any process steps whereby one carries out the lacking process steps, or in which one stops the process at any step, or by which one forms a starting material under the reaction conditions, or in which a reaction component possibly in the form of its salt is present.

Thus, one may react an aldehyde of the formula XIX

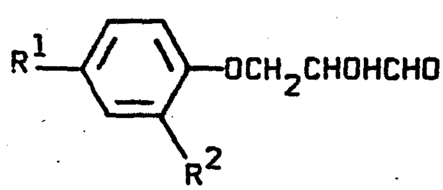

(XIX)

wherein $R^1$ and $R^2$ have the same meanings as given above, with an amine of the formula

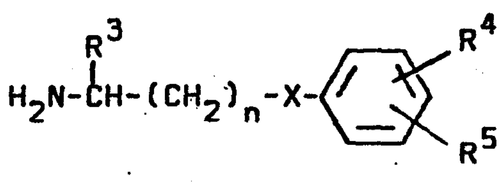

wherein $R^3$, $R^4$, $R^5$, n, and X have the same meanings as given above, in the presence of a suitable reducing agent, such as one of the above mentioned. Thereby a compound of formula VIII is obtained as an intermediate, which then is reduced according to the invention.

Further, one may in a manner known per se react an amine of the formula III with an aldehyde or a ketone of the formula

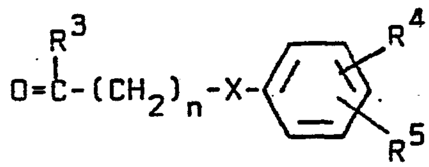

in the presence of a suitable reducing agent, such as one of the above mentioned to produce compounds of formula IX or X as an intermediate, which then is reduced according to the invention.

The new compounds may, depending on the choice of starting materials and process, be present as optical antipodes or racemate, or, if they contain at least two asymmetric carbon atoms, be present as an isomer mixture (racemate mixture).

The isomer mixtures (racemate mixtures) obtained may, depending on physical-chemical differences of the components, be separated into the two stereoisomerically (diastereomerically) pure racemates, e.g., by means of chromatography and/or fractional crystallization.

The racemates obtained can be separated according to known methods, e.g., by means of recrystallization from an optically active solvent, by means of microorganisms, or by a reaction with optically active acids, forming salts of the compound and separating the salts thus obtained, e.g., by means of the different solubilities of the diastereomeric salts, from which the antipodes may be set free by the action of a suitable agent. Suitable optically active acids are, e.g., the L- and D-forms

of tartaric acid, di-o-tolyltartaric acid, malic acid, mandelic acid, camphorsulfonic acid or quinic acid. Depending on the indication area preferably the more activ part of the two antipodes is isolated.

Suitably, such starting materials are used for carrying out the reactions of the invention, which material leads to groups of end products preferably the desired and particularly to the specifically described and preferred, end products.

The starting materials are known or may, if they are novel, be obtained according to processes known <u>per se</u>.

In clinical use the compounds of the invention are usually administered orally, rectally or by injection in the form of a pharmaceutical preparation, which contains the active component either as free base or as pharmaceutically acceptable, non-toxic acid addition salts, e.g., the hydrochloride lactate, acetate, sulphamate or the like in combination with a pharmaceutically acceptable carrier.

Thus, the mention of the new compounds of the invention refers to either the free amine base or the acid addtion salts of the free base, even if the compounds are generally or specifically described, provided that the context in which such expressions are used, e.g. in the examples, do not correspond with this broad meaning. The carrier may be a solid, semisolid or liquid diluent, or a capsule. These pharmaceutical preparations are a further object of the invention. Usually the amount of active compound is between 0,1 and 99% by weight of the preparation, suitably betwen 0,5 and 20% by weight in preparations for injection and between 2 and 50% by weight in preparations for oral administration.

In the preparation of pharmaceutical preparations containing a compound of the present invention in the form of dosage units for oral administration, the compound elected may be mixed with a solid, pulverulent carrier, such as, e.g., with lactose,

saccharose, sorbitol, mannitol, starch, such as potato starch, corn starch, amylopectin, cellulose derivatives or gelatin, as well as with an antifriction agent, such as magnesium stearate, calcium stearate, polyethylene glycol waxes or the like, and be pressed into tablets. If coated tablets are wanted, the above prepared core may be coated with a concentrated solution of sugar; which solution may contain, e.g., gum arabicum, gelatin, talc, titanium dioxide, or the like. Furthermore, the tablets may be coated with a laquer dissolved in an easily volatile organic solvent, or mixture of solvents. To this coating a dye may be added in order to easily distinguish between tablets with different active compounds or with different amounts of the active compound present.

In the preparation of soft gelatine capsules (pearl-shaped, closed capsules), which consist of gelatine and e.g. glycerine, or in the preparation of similar closed capsules, the active compound is mixed with a vegetable oil. Hard gelatine capsules may contain granules of the active compound in combination with a solid, pulverulent carrier as lactose, saccharose, sorbitol, mannitol, starch (as e.g. potatoe starch, corn starch or amylopectin), cellulose derivatives or gelatine.

Dosage units for rectal administration may be prepared in the form of suppositories, which contain the active substance in a mixture with a neutral fat base, or they may be prepared in the form of gelatine-rectal capsules which contain the active substance in a mixture with a vegetable oil or paraffin oil.

Liquid preparations for oral administration may be present in the form of sirups or suspensions, e.g. solutions containing from about 0,2% by weight to about 20% by weight of the active substance described, wherein the reminder consists of sugar and a mixture of ethanol, water, glycerol and propylene glycol. If desired, such liquid preparations may contain colouring agents, flavouring agents, saccharine, and carboxymethylcellulose as a thickening agent.

Solutions for parenteral administration by injection may be prepared as an aqueous solution of a water soluble pharmaceutically acceptable salt of the active compound, preferably in a concentration from about 0,5% by weight to about 10% by weight. These solutions may also contain stabilizing agents and/or buffering agents and may suitably be available in different dosage unit ampoules.

The preparation of pharmaceutically acceptable tablets for peroral use is carried out in accordance with the following method:

The solid substances included are ground or sieved to a certain particle size. The binding agent is homogenized and suspended in a certain amount of solvent. The therapeutic compound and necessary auxiliary agents are mixed with continuous and constant mixing with the binding agent solution and are moistened so that the suspension is uniformly dispersed throughout the mass without overmoistening any parts. The amount of solvent is usually so adjusted that the mass obtains a consistency reminiscent of wet snow. The moistening of the pulverulent mixture with the binding agent solution causes the particles to aggregate slightly and the granulating process is carried out in such a way that the mass is pressed through a sieve in the form of a mesh of stainless steel having a mesh size of about 1 mm. The mass is then placed in thin layers on a tray to be dried in a drying cabinet. This drying takes place during 10 hours and has to be standardized carefully since the dampness of the granulate is of utmost importance for the following process and for the feature of the tablets. Drying in a fluid bed may possibly be used. In this case the mass is not put on a tray but is poured into a container having a mesh bottom.

After the drying step the granules are sieved so that the desired particle size is obtained. Under certain circumstances powder has to be removed.

To this penultimate mixture, disintegrating, antifriction agents and antiadhesive agents are added. After this mixture the mass shall have the proper composition for the tabletting step.

The cleaned tablet punching machine is provided with a certain set of punches and dies, whereupon the suitable adjustment for the weight of the tablets and the degree of compression is tested out. The weight of the tablet is decisive for the size of the dose in each tablet and is calculated starting from the amount of therapeutic agent in the granules. The degree of compression affects the size of the tablet, its strength and its ability of disintegrate in water. Especially with regard to the two later properties the choice of compression pressure (0,5 to 5 ton) requires a compromise. When the right adjustment is set, the preparation of tablets is started and is carried out at a rate of 20,000 to 200,000 tablets per hour. The pressing of the tablets requires different times and depends on the size of the batch.

The tablets are freed from adhering pulver in a specific apparatus and are then stored in closed packages until they are delivered.

Many tablets, especially those which are rough or bitter, are given a coating. This means that they are coated with a layer of sugar or some other suitable coating.

The tablets are usually packed by machines having an electronic counting device. The different types of packages consist of glass or plastic gallipots but also boxes, tubes and specific dosage adapted packages.

The daily dose of the active substance varies and is dependent on the type of administration, but as a general rule it is 100 to 1000 mg/day of active substance for peroral administration and 5 to 1000 mg/day for intravenous administration.

The following Examples illustrate the principle and the adaption of the invention without, however, being limited thereto. Temperature is given in degrees Celsius.

Example 1

Preparation of 3-[2-phenethylamino]-1-[4-(2-methoxyethyl)-phenoxy]-propanol-2

11,5 g of 1,2-epoxy-3-[4-(2-methoxyethyl)phenoxy]-propane were mixed with 6,7 g of 2-phenethylamine and 50 mls of isopropanol and the total solution was refluxed for 3 hours. The solution was thereupon evaporated *in vacuo*. The base thus obtained was dissolved in acetone and the hydrochloride was precipitated using HCl in ether. The hydrochloride was filtered off and washed with acetonitril. The yield of 3-[2-phenethylamino]-1--[4-(2-methoxyethyl)phenoxy]-propanol-2 · HCl was 12,5 g. Melting point 162°C (HCl). The structure was verified using NMR.

Example 2

3-[2-(4-hydroxyphenyl)-ethylamino]-1-[4-(2-methoxyethyl)-phenoxy]-propanol-2 was prepared according to Example 1 using 1,2-epoxy-3-[4-(2-methoxyethyl)phenoxy]-propane and 2-(4-hydroxyphenyl)-ethylamine as starting materials. The melting point of its hydrochloride is 160°C. Its structure was verified by NMR and equivalent weight.

Example 3

3-[2-phenethylamino]-1-[4-(2-methoxyethyl)-2-bromophenoxy]--propanol-2 was prepared according to Example 1 using 1,2--epoxy-3-[4-(2-methoxyethyl)-2-bromophenoxy]-propane and 2-phenethylamine as starting materials. The melting point of the tartrate is 139°C. Its structure was verified by NMR and equivalent weight.

Example 4

3-[1-methyl-2-(4-hydroxyphenyl)-ethylamino]-1-[4-(2-methoxyethyl)-2-bromophenoxy]-propanol-2 was prepared according to Example 1 using 1,2-epoxy-3-[4-(2-methoxyethyl)-2-bromophenoxy]-propane and 1-methyl-2-(4-hydroxyphenyl)-ethylamine as starting material. The hydrochloride melted at 159°C.

Example 5

3-[1-methyl-2-(4-hydroxyphenyl)-ethylamino]-1-[4-(2-methoxyethyl)-2-chlorophenoxy]-propanol-2 was prepared according to Example 1 using 1,2-epoxy-3-[4-(2-methoxyethyl)-2-chlorophenoxy]-propane and 1-methyl-2-(4-hydroxyphenyl)-ethylamine as starting materials. The hydrochloride melted at 150°C.

Example 6

3-phenylmethylamino-1-[4-methoxymethylphenoxy]-propanol-2 was prepared according to Example 1 using 1,2-epoxy-3-(4--methoxymethylphenoxy)-propane and phenylmethylamine as starting materials. The hydrochloride melted at 170.5-171.5°C.

Example 7

3-(2-phenethylamino)-1-[4-methoxymethylphenoxy]-propanol-2 was prepared according to Example 1 using 1,2-epoxy-3-(4--methoxymethylphenoxy)-propane and 2-phenethylamine as starting materials. The hydrochloride melted at 169-169.5°C.

Example 8

3-(4-phenylbutylamino)-1-(4-methoxymethylphenoxy)-propanol-2 Was prepared according to Example 1 using 1,2-epoxy-3-(4--methoxymethylphenoxy)-propane and 4-phenylbutylamine as starting materials. The hydrochloride melted at 154-155°C.

Example 9

3-phenylmethylamino-1-[4-(2-methoxyethyl)phenoxy]-propanol-2 was prepared according to Example 1 using 1,2-epoxy-3-[4-(2--methoxyethyl)phenoxy]-propane and phenylmethylamine as starting materials. The hydrochloride melted at 162.5-164°C.

Example 10

3-(3-phenylpropylamino)-1-[4-(2-methoxyethyl)phenoxy]-propanol-2 was prepared according to Example 1 using 1,2-epoxy-3-[4-(2-methoxyethyl)phenoxy]-propane and 3-phenylpropylamine as starting materials. The hydrochloride melted at 156°C.

Example 11

3-(4-phenylbutylamino)-1-[4-(2-methoxyethyl)phenoxy]-propanol-2 was prepared according to Example 1 using 1,2-epoxy-3-[4-(2-methoxyethyl)phenoxy]-propane and 4-phenylbutylamine as starting materials. The hydrochloride melted at 143-143.5°C.

Example 12

3-[2-(3,4-dimethoxyphenyl)ethylamino]-1-[4-(2-methoxyethyl)phenoxy]-propanol-2 was according to Example 1 using 1,2-epoxy-3-[4-(2-methoxyethyl)phenoxy]-propane and 2-(3,4-dimethoxyphenyl)ethylamine as starting materials. The hydrochloride melted at 124°C.

Example 13

3-[2-phenoxyethylamino]-1-[4-(2-methoxyethyl)phenoxy]-propanol-2 was prepared according to Example 1 using 1,2-epoxy-3-[4-(2-methoxyethyl)phenoxy]-propane and 2-phenoxyethylamine as starting materials. The base melted at 83°C.

Example 14

3-(2-phenethylamino)-1-[4-(2-methoxyethyl)phenoxy]-propanol-2 was prepared according to Example 1 using 1,2-epoxy-3-[4-(2-methoxyethoxy)phenoxy]-propane and 2-phenethylamine as starting materials. The hydrochloride melted at 174°C.

Example 15

3-[4-(3,4-dimethoxyphenyl) butylamino]-1-[4-(2-methoxyethyl)phenoxy]-propanol-2 was prepared according to Example 1 using 1,2-epoxy-3-[4-(2-methoxyethyl)phenoxy]-propane and 4-(3,4-dimethoxyphenyl)butyl amine as starting materials.

Example 16

Synthesis of 3-(p-benzyloxyphenyl)-1-propanol

To a solution of potassium p-(3-hydroxypropyl)-phenoxide, made from 123.1 g (0.809 mol) of 3-(p-hydroxyphenyl)-1-propanol and 56.4 g of potassium hydroxide, in 700 ml acetonitrile was added 151.8 g (1.20 mol) of benzylchloride. After heating the mixture under reflux and filtering off inorganic salts, the solvent and excess benzylchloride were distilled under vacuum, with xylene used as a "chaser". The residual (solidifying) oil weighed 198 g (101%) and contained some xylene but no benzylchloride.

Synthesis of benzyl p-(3-methoxypropyl)phenyl ether

To a solution of lithium 3-(p-benzyloxyphenyl)-1-propoxide, made from 187 g (0.773 mol) of 3-(p-benzyloxyphenyl)-1-propanol and 9.35 g (1.18 mol) lithium hydride, in 935 ml dimethylformamide was added with cooling 298.7 g (2.104 mol) of methyl iodide. After stirring one hour at 25°, another 74 g (0.52 mol) of methyl iodide were added, and the mixture was heated to 120° for three hours. One-fourth of this mixture was poured into 1636 ml of water; three 234 ml portions of water were used to rinse. Extraction of the aqueous phase with three 467 ml portions of hexane, washing of the hexane with two 467 ml portions of water, drying, and distillation of the solvent gave 41.5 g of yellow oil. The oil, dissolved in 41.5 ml hexane, was passed through a 2.5 X 25 cm alumina column and eluted with 330 ml hexane. Distillation of the solvent gave 33 g (66.7%) of oil. The remaining three quarters of the batch were treated similarly to give 146 g (73.8%). Boiling point: 142-143° 0.1 mm Hg.

Synthesis of p-(3-methoxypropyl)phenol

An 18.34 g portion of benzyl p-(3-methoxypropyl)phenyl ether was debenzylated in ethanol over 5% palladium on charcoal catalyst under 7-8 atmospheres of hydrogen pressure at 47°. After removal of the catalyst and solvent, there remained 10.0 g (84%) of tan oil.

Synthesis of p-(3-methoxypropyl)phenyl glycidyl ether

To a solution of potassium p-(3-methoxypropyl)phenoxide, made from 3.96 g (0.060 mol) of potassium hydroxide and 10.0 g (0.060 mol) of p-(3-methoxypropyl)phenol, in 71 ml of ethanol was added 23.1 g (0.250 mol) of epichlorohydrin. After stirring at 25$^{o}$ for 18 hours. the suspension was filtered, and solvent and excess epichlorohydria were distilled off under vacuum, leaving 13.1 g (98.5%) of amber oil.

Synthesis of 1-(2-phenylethylamino)-3-[4-(3-methoxy-propyl) phenoxy]-2-propanol

A solution of 1.0 g (0.0045 mol) of p-(3-methoxypropyl)phenyl glycidyl ether and 0.700 g (0.0059 mol) 2-phenylethylamine in 10 ml 2-propanol was heated under reflux for 2.5 hours. After distilling away the solvent, the residual oil was taken up in ether and dilute hydrochloric acid was added to pH 8. The ethereal phase was separated and treated with more hydrochloric acid to pH 2.4. The hydrochloride of the product precipitated from this mixture and was filtered. Yield: 0.700 g (41.0%). Recrystallization from acetonitrile gave white plate-like crystals, mp. 167.5-168.5$^{o}$.

Example 17

A syrup containing 2% (weight per volume) of active substance was prepared from the following ingredients:

| | | |
|---|---|---|
| 3-[2-(4-hydroxyphenyl)-ethylamino]-1-[4-(2-methoxyethyl)phenoxy]-propanol-2 · HCl | | 2.0 g |
| Saccharine | | 0.6 g |
| Sugar | | 30.0 g |
| Glycerine | | 5.0 g |
| Flavouring agent | | 0.1 g |
| Ethanol 96% | | 10.0 g |
| Distilled water | ad | 100.0 ml |

Sugar, saccharine and the salt of the active substance were dissolved in 60 g of warm water. After cooling, glycerine and solution of flavouring agents dissolved in ethanol were

added. To the mixture water was tnen added to 100 ml.

The above named active substance may be replaced by other pharmaceutically acceptable acid addition salts.

Example 18

3-[2-(4-hydroxyphenyl)-ethylamino]-1-[4-(2-methoxyethyl)-phenoxy]-propanol-2 hydrochloride (250 g) was mixed with lactose (175,8 g), potatoe starch (169,7 g) and colloidal silicic acid (32 g). The mixture was moistened with a 10% solution of gelatine and was granulated through a 12-mesh sieve. After drying potatoe starch (160 g), talc (50 g) and magnesium stearate (5 g) were admixed and the mixture thus obtained was pressed into tablets (10,000), each containing 25 mg of active substance. The tablets are sold on the market provided with a breaking score to give another dose than 25 mg or to give multiples thereof when broken.

Example 19

Granules were prepared from 3-[2-(4-hydroxyphenyl)-ethylamino]--1-[4-(2-methoxyethyl)phenoxy]-propanol-2-p-hydroxybenzoate (250 g), lactose (175,9 g) and an alcoholic solution of polyvinylpyrrolidone (25 g). After the drying step the granules were mixed with talc (25 g), potatoe starch (40 g) and magnesium stearate (2,50 g) and were pressed into 10,000 tablets being biconvex. These tablets are coated with a 10% alcoholic solution of shellac and thereupon with an aqueous solution containing saccharose (45%), gum arabicum (5%), gelatine (4%) and dyestuff (0,2%). After the first five coatings talc and powdered sugar were used for powdering. The priming coat was then coated with a 66% sugar syrup and polished with a 10% carnauba wax solution in carbon tetrachloride.

Example 20

3-[2-(4-hydroxyphenyl)-ethylamino]-1-[4-(2-methoxyethyl)-phenoxy]-propanol-2-hydrochloride (1 g), sodium chloride (0,8 g) and ascorbic acid (0,1 g) were dissolved in sufficient amount of distilled water to give 100 ml of solution. This solution, which contains 10 mg of active substance in each ml, was used in filling ampoules, which were sterilized by heating at 120°C for 20 minutes.

Example 21 (method b + e)

10 g of 4-(2-methoxyethyl)phenyl glycidylether in 100 ml of ethanol were saturated with gaseous ammonia and the mixture was heated in an autoclave on a boiling waterbath for 4 hours. The solvent was evaporated, the residue was dissolved in ethyl acetate and HCl-gas was introduced. The hydrochloride then precipitated and it was filtered off and dissolved in 50 ml of ethanol to which 2-(4-methoxymethoxy-phenyl)-ethylchloride and 15 g of $K_2CO_3$ had been added. The mixture was heated in an autoclave at 130°C for 10 hours whereupon the solvent was evaporated and the residue was treated with 100 ml of 2N HCl for 1 h at ambient temperature. The aqueous phase was made alkaline with ammonia and extracted with ethyl acetate. The solvent phase was dried over $K_2CO_3$, whereupon 3-[2-(4--hydroxyphenyl)-ethylamino]-1-[4-(2-methoxyethyl)phenoxy]-propanol-2 was obtained. The base obtained was converted to its hydrochloride, 3-[2-(4-hydroxyphenylethylamino]-1-[4-(2--methoxyethyl)phenoxy]-propanol-2 · hydrochloride. Melting point 160°C.

Example 22

2,4 g of Na were dissolved in 100 ml of ethanol, whereupon 10,8 g of 4-(2-methoxyethyl)phenol and then 22,9 g of 1-[2-(4--methoxymethoxyphenyl)-ethylamino]-3-chloropropanol-2 were added. The mixture was heated in an autoclave on a boiling waterbath for 10 hours. Thereupon it was filtered and the filtrate was evaporated to dryness. The residue was treated with 2N HCl for 1 h at ambient temperature and extracted with ether, whereupon the aqueous phase was made alkaline with ammonia and extracted with ether. The ether phase was dried over $MgSO_4$ and 3-[2-(4-hydroxyphenyl)-ethylamino]-1-[4-(2--methoxyethyl)phenoxy]-propanol-2 was obtained and was converted to its hydrochloride and isolated. Melting point 160°C.

Example 23 (method d)

0,116 moles of 4-(2-methoxyethyl)-phenol were mixed with 0,080 moles of 1-[2-(4-methoxymethoxyphenyl)ethyl]-3-azetidinol, 0,500 moles of benzylalcohol and 0,003 moles of KOH. The

mixture was refluxed while stirring for 6 hours at 140°C and was then cooled and extracted with 2N HCl. The aqueous phase was allowed to stand for 1 hour at ambient temperature, was then made alkaline, and was finally extracted with chloroform. After drying and evaporation the residue was dissolved in ether and to the solution HCl in ether was added. The hydrochloride was filtered off and was washed with acetone. The hydrochloride of 3-[2-(4-hydroxyphenyl)ethylamino]-1-[4-(2--methoxyethyl)phenoxy]-propanol-2 melted at 160°C.

Example 24 (method f)

In accordance with Example 11 above, 1-amino-3-[4-(2-methoxyethyl)phenoxy]-propanol-2 was prepared. 5 g of this compound were dissolved in 50 ml of methanol and 15 g of 4-hydroxyphenyl-acetaldehyde were added, whereby 3-[2-(4-hydroxyphenyl)-ethylimino]-1-[4-(2-methoxyethyl)phenoxy]-propanol-2 was obtained. The solution was cooled to 0°C and at this temperature 5 g of sodium borohydride were added little by little, whereby the imini compound was reduced. The temperature was then allowed to rise to ambient temperature and after 1 hour 150 ml of $H_2O$ were added and the total mixture was extracted with ether. The ether phase was dried over $MgSO_4$ and was evaporated. The residue was transformed into its hydrochloride. In this way 3-[2-(4-hydroxyphenyl)-ethylamino]-1-[4-(2-methoxyethyl)phenoxy]-propanol-2 · HCl was obtained. Melting point 160°C.

Example 25 (method g)

1,0 g of 3-[4-(2-methoxyethyl)phenoxy]-1-[2-(4-hydroxyphenyl)-ethylamino]-propanone-2 was dissolved in 25 ml of methanol and the solution was cooled to 0°C on an ice-bath. 0,25 g of $NaBH_4$ were added little by little while stirring first at 0°C for 1 hour and then at ambient temperature for 0,5 hour. The solution thus obtained was evaporated, whereupon 50 ml of $H_2O$ were added. The aqueous phase was extracted 3 times with 50 ml chloroform, the collected chloroform phase was dried and evaporated. The hydrochloride was precipitated from an ether solution of the residue by adding ether containing HCl. Recrystal-

lization was made from acetone. The hydrochloride of 3-[2-(4-hydroxyphenyl)-ethylamino]-1-[4-(2-methoxyethyl)-phenoxy]-propanol-2 melted at 160°C.

Example 26 (method h)

3-[2-phenethylamino]-1-[4-(2-chloroethyl)-phenoxy]-propanol-2 was prepared in accordance with Example 1 above from 1,2--epoxy-3-[4-(2-chloroethyl)phenoxy]propane and 2-phenethylamine. The calculated amount of sodium was dissolved in methanol and 3-(2-phenethylamino)-1-[4-(2-chloroethyl)phenoxy]-propanol-2 was carefully added. After filtration the filtrate was evaporated to dryness and the residue was recrystallized from isopropanol. In this way 3-[2-phenethylamino]-1-[4-(2--methoxyethyl)phenoxy]-propanol · HCl was obtained.

Example 27 (method h)

4,4 g of sodium were added to 270 mls of ethanol. At ambient temperature 36,0 g of 4-(1´,1´-diethoxy-2´-methoxyethyl)phenol were added to the solution. 55 mls of epichlorohydrine were added and the reaction mixture was stirred for 24 hrs. The reaction mixture was evaporated to dryness. The residue was dissolved in chloroform and washed with distilled water. The chloroform phase was dried over sodium sulphate and was evaporated. The residue was dissolved in 200 ml isopropanol and 20 g of 2-phenethylamine were added. The mixture was refluxed for 5 hrs. The reaction mixture was then evaporated and the residue was dissolved in water which was made acidic to pH 1 using conc. hydrochloric acid. The mixture was stirred for 1 hour in order to break down the ketal. The mixture was then extracted with ethyl acetate. The aqueous phase was made alkaline and was extracted with ethyl acetate twice. The collected ethyl acetate phases were treated with active carbon, dried and evaporated.

The hydrochloride was isolated by precipitating it from HCl--isopropanol by adding ethyl acetate.

20 g of 1-(2-phenethylamino)-3-[4-(2-methoxy-1-oxo-ethyl)-phenoxy]-propanol-2 hydrochloride were dissolved in 200 ml of acetic acid. 1,5 g of 10% Pd/C were added and hydrogenation took place at 70°C and 5 atm until theoretical amount of hydrogen had been absorbed. The catalyst was filtered off and the solvent was evaporated. The residue was dissolved in water and the solution was made alkaline using NaOH. The product was extracted with chloroform and the chloroform was evaporated. The hydrochloride was isolated. M.p. 132°C. Yield 18 g.

Biological effects

The β-receptor blocking agents of the present invention were tested as regards to their biological properties. All compounds were thereby tested in anesthetized cats (males and females weighing 2,5-3,5 kg) pretreated with reserpine (5 mg/kg bodyweight administered intramusculary) about 16 hours before the experiments. The animals were pretreated with reserpine in order to eliminate the endogenous sympathetic control of heart rate and vascular smooth muscle tone. The cats were anesthetized with pentobarbital (30 mg/kg bodyweight administered i.p.) and artificially ventilated with room air. A bilateral vagotomy was performed in the neck. Blood pressure was obtained from a cannulated carotid artery and heart rate was registered from a cardiotachometer, triggered by the electrocardiogram (ECG). The test compounds were given intravenously in increasing doses. The values obtained were plotted as dose-response curves, from which $ED_{50}$ values were estimated. At the end of each experiment high doses of isoprenaline were given in order to obtain the maximal heart rate response.

The experiments demonstrate that the compounds tested are cardioselective β-receptor antagonists.

The $\beta_1$-blocking activity is expressed as $ED_{50}$ (mg/kg) for blockade of isoprenaline-induced tachycardia in anesthetized and reserpinized cats.

The $\beta_2$-blocking activity is expressed as $ED_{50}$ (mg/kg) for blockade of the decreasing effect of isoprenaline on the peripheral vascular resistance in the hind-leg of anesthetized and reserpinized cats.

Acute intravenous toxicity in mice (male) weighing 20-25 g was determined as $LD_{50}$ i.v. in mg/kg:

Membrane stabilizing activity was determined in conscious guinea-pigs after intracutaneous injections, (according to E. Bülbring & J. Wajda, J. Pharmacol. Exp. Therap. 1945, 85, 78-84, whereby the effects obtained were compared to that of propranolol. Effect equal to that of propanolol = 2, effect not as good as that of propanolol = 1, no effect = 0, effect better than that of propranolol = 3. Results obtained in above given tests are given in Table 1 below.

Table 1

| Compound | $LD_{50}$ i.v. mg/kg | $\beta_1$-blockade of isoprenaline on heart rate $ED_{50}$ mg/kg | $\beta_2$-blockade of isoprenaline on peripheral resistance $ED_{50}$ mg/kg | Membrane stabilizing activity |
|---|---|---|---|---|
| Propranolol | ~35 | 0,1 | 0,1 | 2 |
| Alprenolol | ~35 | 0,05 | 0,05 | 2 |
| Practolol | 100 | 0,3 | 35 | 0 |
| Metoprolol | 70 | 0,3 | 4,7 | 1 |
| D-Propranolol | - | ~ 5 | ~ 5 | ~ 2 |
| Lidocaine | 30 | - | - | 2 |
| Ex. 1 | ~40 | 4,8 | ≫10 | 2 |
| Ex. 2 | >50 | 1,05 | 12,8 | 2 |
| Ex. 3 | ~90 | 0,5 | > 8,5 | 2 |
| Ex. 4 | <100 | 0,8 | 5,9 | 2 |
| Ex. 5 | 50-100 | 0,8 | > 8,5 | 2 |
| Ex. 9 | 57 | 15 | >22 | |
| Ex. 14 | 52 | 5 | >>10 | |
| Ex. 10 | 32 | 4 | >> 6 | |
| Ex. 11 | 39 | >6 | > 6 | |
| Ex. 6 | 64 | | | |
| Ex. 7 | ~55 | | | |
| Ex. 15 | | 1.6 | >6.6 | |

I. The antiarrhythmic activities of the compounds have been tested against ouabain-induced arrhythmias in anesthetized guinea-pigs.

Method

Guinea-pigs of either sex (300-400 g) were anesthetized with urethane 1,4 g/kg i.p. and tracheotomized. ECG, lead II was picked up with needle electrodes and was registered continuously throughout the experiment. Two i.v. catheters were inserted: one for the administration of ouabain, the other for the test solution.

The experimental design for the study of antiarrhythmic activities is schematically shown in the head of Table 2. The arrhythmia was induced by automatic infusion of ouabain, 18 µg/kg/60" every two minutes for twelve minutes and thereafter 9 µg/kg/30" every two minutes throughout the whole experiment and the infusion was not stopped until asystole occurred. As soon as the frequency of ventricular premature beats (VPB) had passed 50 per cent, the test solution was given i.v. (0,2 ml/min) during 2 min. (see head of Table 2). In experiments where the test solution contained an antiarrhythmic substance, the frequency of premature ventricular beats was then reduced, but since administration of ouabain continued, the frequency of ectopics increased again. After some time the arrhythmia was converted into bursts of ventricular fibrillation (V-FIB) and finally the animal died.

Result

The results are shown in Table 2, from which it is evident that the new compounds are extremely potent as antiarrhythmic compounds, although their $\beta_1$-blocking activities intentionally have been made considerably weaker than that of, e.g., propranolol. Thus, looking at the β-blocking potency (cf Table 1) of the new compounds it is evident that they exert unexpectedly strong antiarrhythmic effects.

Table 2

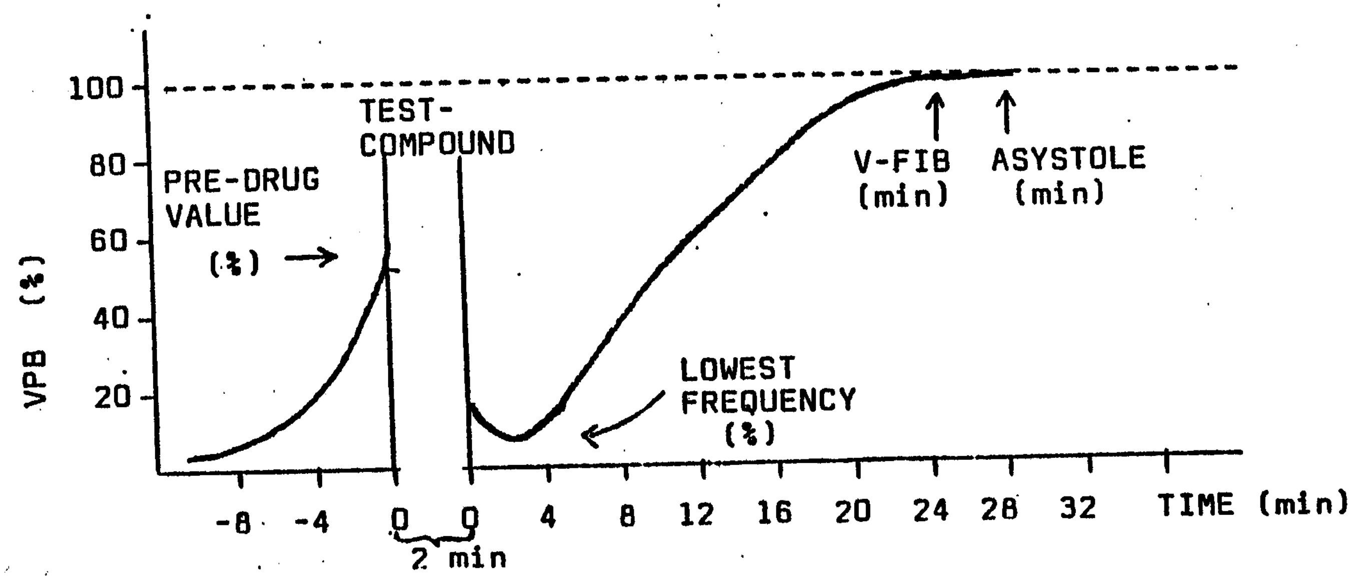


| TEST COMPOUND | DOSE mg/kg | NO OF ANIMALS | PRE-DRUG VALUE % | LOWEST FREQUENCY % | A-A EFFECT IN NUMBER OF ANIMALS | V-FIB min | ASYSTOLE min |
|---|---|---|---|---|---|---|---|
| PROPRANOLOL | 0.03 | 7 | 60±4 | 29±5 | 6/7 | 21±3 | 29±2 |
| " | 0.1 | 7 | 58±2 | 9±5 | 6/7 | 30±5 | 35±4 |
| " | 0.3 | 6 | 62±5 | 4±2 | 6/6 | 27±6 | 32±5 |
| D-PROPRANOLOL | 0.3 | 8 | 57±4 | 36±5 | 6/8 | 14±1 | 22±1 |
| " | 1.0 | 6 | 56±3 | 5±3 | 6/6 | 23±2 | 29± [illegible] |
| METOPROLOL | 0.03 | 6 | 59±2 | 34±5 | 6/6 | 25±3 | 35±4 |
| " | 0.1 | 6 | 61±6 | 16±7 | 6/6 | 32±5 | 38±5 |
| " | 0.3 | 6 | 63±4 | 1±1 | 6/6 | 37±3 | 48±6 |

Table 2 (cont.)

| TEST COMPOUND | DOSE mg/kg | NO OF ANIMALS | PRE-DRUG VALUE % | LOWEST FREQUENCY % | A-A EFFECT IN NUMBER OF ANIMALS | V-FIB min | ASYSTOLE min |
|---|---|---|---|---|---|---|---|
| EX 4 | 0.3 | 6 | 58±2 | 31±14 | 3/6 | 19±1 | 27±1 |
| " | 1.0 | 6 | 60±3 | 6±3 | 6/6 | 31±3 (N=4) | 41±3 |
| EX 3 | 0.3 | 6 | 58±1 | 26±7 | 5/6 | 27±1 | 34±1 |
| " | 1.0 | 6 | 57±4 | 13±7 | 6/6 | 35±2 | 41±2 |
| EX 2 | 0.1 | 7 | 58±3 | 25±7 | 7/7 | 20±2 | 27±3 |
| " | 0.3 | 6 | 61±4 | 5±2 | 6/6 | 29±2 | 35±3 |
| EX 1 | 0.3 | 8 | 57±1 | 38±13 | 4/8 | 19±1 | 28±1 |
| " | 1.0 | 9 | 61±3 | 9±4 | 9/9 | 31±4 | 38±4 |
| LIDOCAINE | 4.0 | 5 | 59±6 | 10±1 | 5/5 | 15±2 | 21±1 |
| " | 12.0 | 6 | 55±4 | 5±3 | 5/6 | 22±3 | 30±3 |
| NaCl 0.9 % | - | 8 | 59±2 | - | 0/8 | 16±3 | 21±4 |

The antiarrythmic effect of some of the compounds has also been tested on dogs provided with a ligation of a coronary artery according to Harris (Circulation, 1950, 1, 1318-1328).

Method

Male beagle dogs, 7-13 kg, undergo two days prior to the test a surgical ligation of a coronary artery. On the day of the test the conscious dog is secured in a canvas sling in a standing position and given increasing doses of the test compounds by intravenous administration. ECG, blood pressure etc are monitored and attention is paid to all CNS-effects. The dogs have almost 100 percent of ectopics prior to administration of the compound to be tested. Effects of the test drug on the frequency of ectopics are calculated from ECG. The accumulative end doses giving 50% and 90% reduction of ectopics as well as the $ED_{50}$ values which give convulsions (side-effect) are measured and given below in Table 3. The value for convulsions is a mean value for 3-5 dogs.

Table 3

| Compound | Conscious dog Harris ligation Ectopics -50% mg/kg iv. | -90% mg/kg iv. | $ED_{50}$ mg/kg iv. Convulsive |
|---|---|---|---|
| Propranolol | 1 | 3 | 8 |
| D-Propranolol | 1 | 4 | 8 |
| Practolol | >16 | >16 | >16 |
| Metoprolol | 4 | 8 | >8 |
| Tocainide | 4 | 8 | >8 |
| Ex. 6 | 8 x) | >16 | 14 |
| Ex. 7 | 4 | 8 | >8 |
| Ex. 8 | 4 | >8 | 8 |
| Ex. 9 | 2 | 4 | 8 |
| Ex. 14 | 1 | 2 | 6-8 |
| Ex. 10 | 2 | 4 | >8 |
| Ex. 11 | 1 | 2 | 8 |
| Ex. 2 | 4 | 8 | 8 |
| Ex. 13 | 4 | 4 | 8 |
| Ex. 15 | 1 | 4 | 6 |

x) The value given is the final value in a series of doses 0.5 + 1 + 2 + 4 + 8 mg/kg i.v. giving 50% reduction of ectopics. 30 min interval between doses.

Table 3: Antiarrythmic effect and CNS-effects of four β-receptor blockers, a membrane stabilizer, and ten new compounds with combined β-blocking and membrane stabilizing effect.

II. The antiarrhythmic effect of some of the compounds has also been tested against chloroform-induced ventricular arrhythmias in mice.

Method

Mice were given an intraperitoneal dose of the test compound. Twenty minutes after dose, they were suffocated in chloroform vapor. When the animals stopped breathing, the chest was opened to inspect for fibrillation. If the heart was not fibrillating, it was prodded. If fibrillation did not occur despite the prodding, the animals were "protected", against fibrillation.

Signs of CNS toxicity were noted during the 20-minute interval between dosing and challenge.

As shown in Table 4, the new compounds tested exerted favourable antiarrhythmic effects also in this test.

Table 4

| Substance | Dose mg/kg | Number of Animals | | | | |
|---|---|---|---|---|---|---|
| | | Treated | Protected | Convulsive | LRR | Dead |
| Propranolol | 100 | 10 | 10 | 10 | 1 | 0 |
| Pamatolol x) | 100 | 10 | 5 | 0 | 0 | 0 |
| Lidocaine | 100 | 10 | 5 | 10 | 10 | 0 |
| Ex. 1 | 100 | 10 | 9 | 1 | 0 | 0 |
| Ex. 2 | 100 | 10 | 8 | 0 | 0 | 0 |

x) Pamatolol is a selective $\beta_1$-receptor blocking agent without membrane stabilizing activity.

Table 4. Antiarrhythmic effects and CNS effects of two betablockers (propranolol, pamatolol), a membrane stabilizer (lidocaine) and two new compounds with combined ß-blocking and membrane stabilizing activities.

"Protected" means number of animals protected against $CHCl_3$-induced ventricular fibrillation.

"Convulsive" and "LRR" denotes the number of animals that showed convulsions and loss of righting reflex.

In an alternative chloroform test on mice the ventricular tachycardia inhibition by the test compounds are observed. Thereby the test solution is given subcutaneous and the animal is observed for CNS-effects for 20 min. The mouse is put into a chamber with chloroform until it stops breathing (approx 30 sec.). The animal is then secured on a cork-board and ECG is monitored. If the ventricular rythm is less than 520 beats per min., the animal was protected against ventricular tachycardia. Groups of 10 animals were tested for each dose-level and the $ED_{50}$-values have been calculated therefrom. During the test the side-effect ataxia (incordination of movements) is observed. The $ED_{50}$ value giving ataxia is given in the Table 5 as well.

In Table 5, as shown, the new compounds tested exerted antiarrhythmic effects also in this test.

Table 5

| Compound | Ventricular tachycardia inhibition $ED_{50}$ mg/kg sc. | Ataxia $ED_{50}$ mg/kg sc. |
|---|---|---|
| Propranolol | 0.6 | ≫1 |
| D-propranolol | ~8 | ~30 |
| Practolol | 2 | >50 |
| Metoprolol | 2.6 | >50 |
| Tocainide | 90 | 130 |
| Ex. 6 | ~200 | ~70 |
| Ex. 7 | ~50 | ~100 |
| Ex. 9 | 35 | 60 |
| Ex. 14 | 10 | 40 |
| Ex. 10 | 3 | >50 |
| Ex. 11 | 15 | >50 |
| Ex. 2 | 3 | >13 |
| Ex. 13 | 3 | >12 |
| Ex. 15 | 1.2 | >6.3 |

Table 5: Inhibition of chloroform induced ventricular tachycardia and appearing ataxia of four β-receptor blockers, one membrane stabilizer, and nine new compounds having combined β-receptor blocking activity and membrane stabilizing effect.

Summary clauses

1. A process for the preparation of new amines of the formula

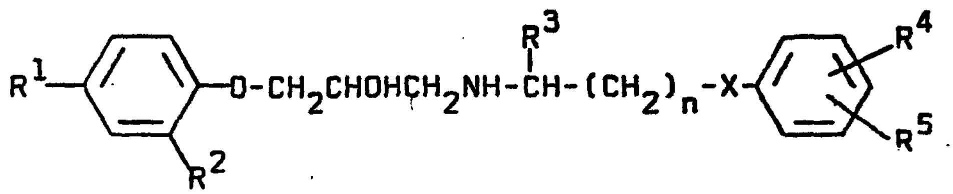

(I)

wherein $R^1$ is selected from the group consisting of alkoxyalkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl and hydroxy, n is an integer 0, 1, 2, or 3, and X is -O-, $\overset{O}{\overset{\|}{C}}$, or $-CH_2-$ , whereby n is not zero, when X is oxygen, characterized in that

a) a compound of the formula II

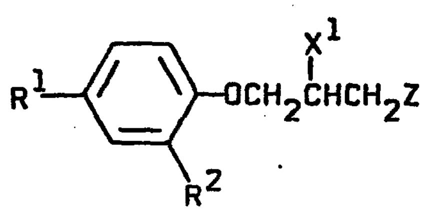

(II)

wherein $R^1$ and $R^2$ have the same meanings given above, $X^1$ is a hydroxy group and Z is a reactive, esterified hydroxy group, or $X^1$ and Z together form an epoxy group, is allowed to react with an amine of the formula

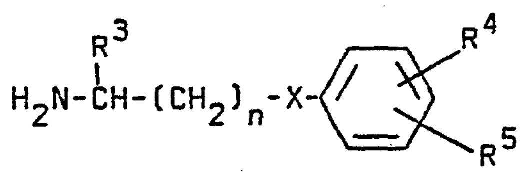

wherein $R^3$, $R^4$, $R^5$, n and X have the same meanings as given above, or

b) a compound of the formula III

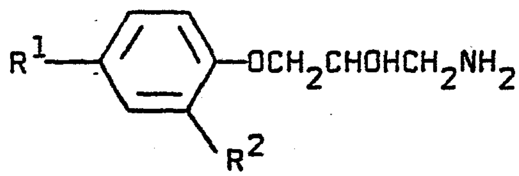

(III)

wherein $R^1$, and $R^2$ have the same meanings as given above, is allowed to react with a compound of the formula

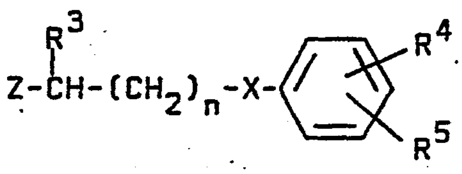

wherein $R^3$, $R^4$, $R^5$, n, X, and Z have the same meanings as given above,

c) a compound of the formula IV

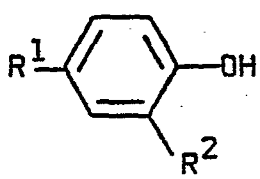

(IV)

wherein $R^1$, and $R^2$ have the same meanings as given above, is allowed to react with a compound of formula V

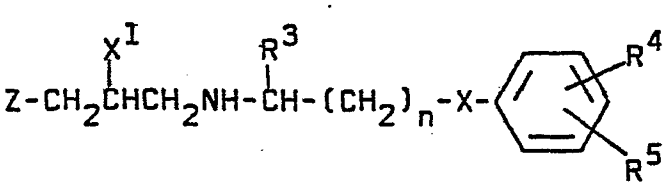

(V)

wherein Z, $X^1$, $R^3$, $R^4$, $R^5$, n, and X have the same meanings as given above, or

d) a compound of the formula IV

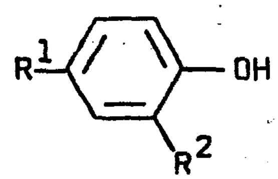

(IV)

wherein $R^1$, and $R^2$ have the same meanings as given above, is allowed to react with a compound of the formula VI

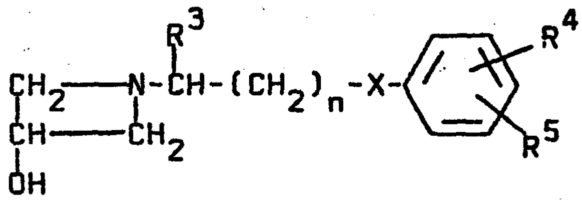

(VI)

wherein $R^3$, $R^4$, $R^5$, n and X have the same meanings as given above,

e) from a compound of the formula I wherein $R^1$, $R^2$, $R^3$, $R^4$, $R^5$, n, and X have the same meanings as above, and which compound has a removable residue on the nitrogen atom of the amino group and/or has a removable residue on the hydroxy groups, this residue is split off,

f) a Schiff's base of the formula VIII or IX

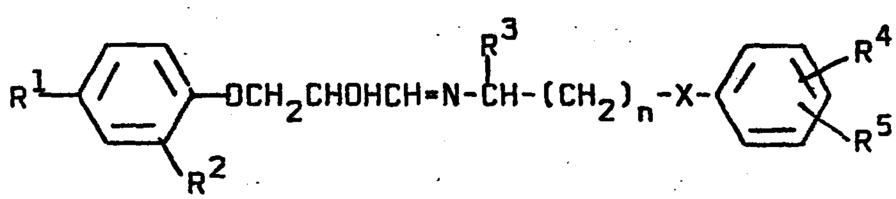

(VIII)

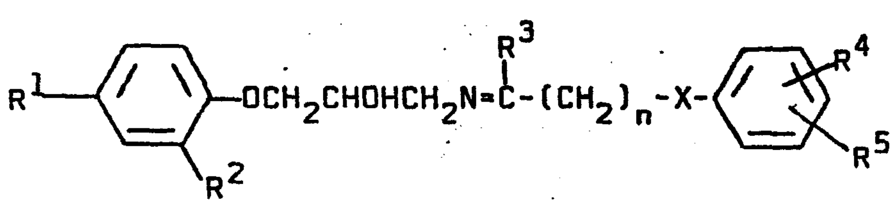

(IX)

or a cyclic tautomer X related to the compound of formula IX

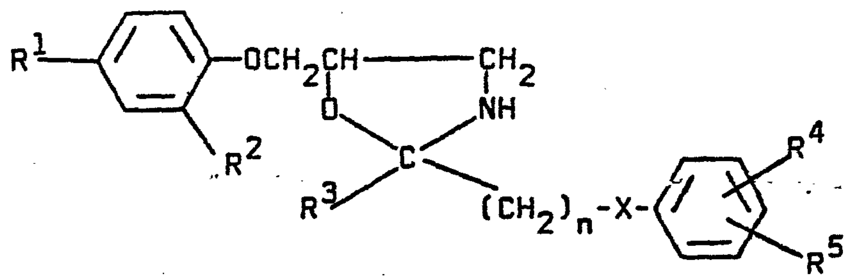

(X)

wherein $R^1$, $R^2$, $R^3$, $R^4$, $R^5$, n, and X have the same meanings as above, except for the case when n = 0 and X = O, whereby the compounds IX and X may be present simultaneously, is reduced, or

g) in a compound of the formula XI

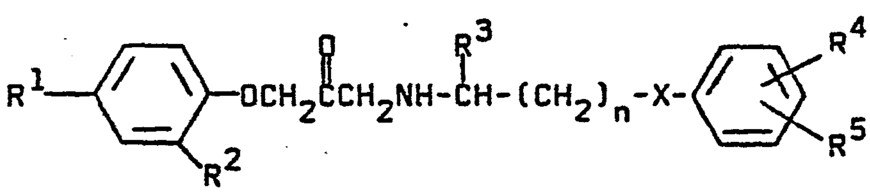

(XI)

wherein $R^1$, $R^2$, $R^3$, $R^4$, $R^5$, n, and X have the same meanings as above, the oxo group is reduced to a hydroxy group,

h) in a compound of the formula XII

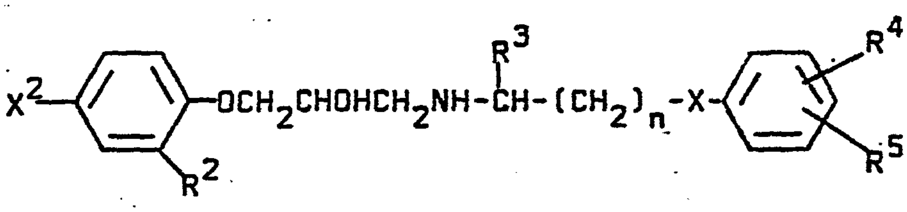

wherein $R^2$, $R^3$, $R^4$, $R^5$, n, and X have the same meanings as above and wherein $X^2$ is a residue transformable into $R^1$ having the above meanings, $X^2$ is transformed into $R^1$, or

i) a compound of formula Va

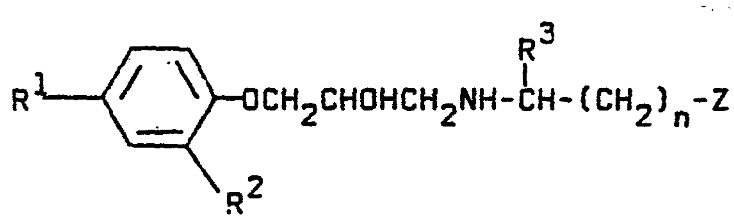

(Va)

wherein $R^1$, $R^2$, $R^3$, n, and Z have the same meanings as above is allowed to react with a compound of formula IVa

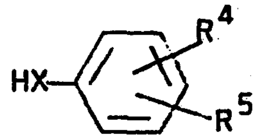

(IVa)

wherein $R^4$, and $R^5$ have the same meanings as above, and X is O; or

k) in a compound corresponding to the one of formula I having an oxo group at a C-atom adjacent to the N-atom, this oxo group is reduced to two hydrogen atoms, and

if desired, isomer mixtures obtained are separated into pure isomers, and/or racemates obtained are separated into optical antipodes, and/or free bases obtained are transformed into their therapeutically acceptable salt, or salts obtained are transformed into their free bases.

2. A process according to clause 1, wherein
1) 3-[2-phenethylamino]-1-[4-(2-methoxyethyl)phenoxy]-propanol-2,
2) 3-[2-(4-hydroxyphenyl)-ethylamino]-1-[4-(2-methoxyethyl)-phenoxy]-propanol-2,
3) 3-[2-phenethylamino]-1-[4-(2-methoxyethyl)-2-bromophenoxy]-propanol-2,
4) 3-[1-methyl-2-(4-hydroxyphenyl)-ethylamino]-1-[4-(2--methoxyethyl)-2-bromophenoxy]-propanol-2,
5) 3-[1-methyl-2-(4-hydroxyphenyl)-ethylamino]-1-[4-(2--methoxyethyl)-2-chlorophenoxy]-propanol-2,
6 ) 3-[2-(4-hydroxyphenoxy)-ethylamino]-1-[4-(2-methoxyethyl)-phenoxy]-propanol-2,
7 ) 3-[2-(4-hydroxyphenoxy)-ethylamino]-1-[4-(2-methoxyethyl)--2-bromophenoxy]-propanol-2,
8 ) 3-[2-(4-hydroxyphenylcarbonyl)-ethylamino]-1-[4-(2-methoxyethyl)phenoxy]-propanol-2,

9) 3-[2-(3,5-dimethoxyphenyl)-ethylamino]-1-[4-(2-hydroxyethyl)phenoxy]-propanol-2,

10) 3-[2-(2-cyano-5-methylphenyl)-ethylamino]-1-(4-ethylphenoxy)-propanol-2,

11) 3-[2-(3,5-diethylphenylcarbonyl)-ethylamino]-1-(4-ethyl-2-bromophenoxy)-propanol-2,

12) 3-(2-phenethylamino)-1-[4-(2-methoxy-1-methylethyl)-phenoxy]-propanol-2,

13) 3-(2-phenethylamino)-1-[4-(methoxymethyl)phenoxy]-propanol-2, or

14) 3-(3-phenylpropylamino)-1-[4-(2-methoxyethyl)phenoxy]-propanol-2,

or its therapeutically acceptable salts is prepared.

3. Compounds of the formula I

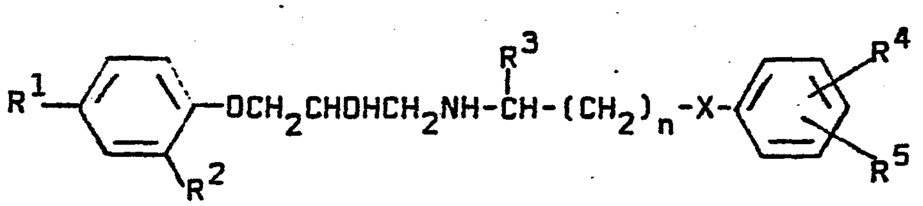 (I)

wherein $R^1$ is selected from the group consisting of alkoxyalkyl, hydroxylalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl, and hydroxy, n is an integer 0, 1, 2, or 3, and X is -O-, $-\overset{O}{\overset{\|}{C}}-$, or $-CH_2-$, whereby n is not zero, when X is oxygen.

4. A compound according to clause 3 in the form of a dextrorotating optical antipode.

5. A compound according to clause 3 in the form of a levorotating antipode.

6. A compound according to any one of clauses 3-5 in the form of the free base.

7. A compound according to any one of clauses 3-5 in the form of a salt.

8. A compound according to any one of clauses 3-5 in the form of a therapeutically acceptable salt.

9. A compound according to clause 3, wherein
1) 3-[2-phenethylamino]-1-[4-(2-methoxyethyl)phenoxy]-propanol-2,
2) 3-[2-(4-hydroxyphenyl)-ethylamino]-1-[4-(2-methoxyethyl)-phenoxy]-propanol-2,
3) 3-[2-phenethylamino]-1-[4-(2-methoxyethyl)-2-bromophenoxy]-propanol-2,
4) 3-[1-methyl-2-(4-hydroxyphenyl)-ethylamino]-1-[4-(2--methoxyethyl)-2-bromophenoxy]-propanol-2,
5) 3-[1-methyl-2-(4-hydroxyphenyl)-ethylamino]-1-[4-(2--methoxyethyl)-2-chlorophenoxy]-propanol-2,
6) 3-[2-(4-hydroxyphenoxy)-ethylamino]-1-[4-(2-methoxyethyl)-phenoxy]-propanol-2,
7) 3-[2-(4-hydroxyphenoxy)-ethylamino]-1-[4-(2-methoxyethyl)--2-bromophenoxy]-propanol-2,
8) 3-[2-(4-hydroxyphenylcarbonyl)-ethylamino]-1-[4-(2-methoxyethyl)phenoxy]-propanol-2,
9) 3-[2-(3,5-dimethoxyphenyl)-ethylamino]-1-[4-(2-hydroxyethyl)phenoxy]-propanol-2,
10) 3-[2-(2-cyano-5-methylphenyl)-ethylamino]-1-(4-ethyl-phenoxy)-propanol-2,
11) 3-[2-(3,5-diethylphenylcarbonyl)-ethylamino]-1-(4-ethyl-2-bromophenoxy)-propanol-2,
12) 3-(2-phenethylamino)-1-[4-(2-methoxy-1-methylethyl)phenoxy]-propanol-2,
13) 3-(2-phenethylamino)-1-[4-(methoxymethyl)phenoxy]-propanol--2, or
14) 3-(3-phenylpropylamino)-1-[4-(2-methoxyethyl)phenoxy]-propanol-2,
or its therapeutically acceptable salt.,

10. The method for treating symptoms and signs of arrhythmic conditions by stabilizing membranes of the heart and/or by blocking β-receptors of the heart by administering to mammals, including man, suffering from symptoms and signs of arrhythmic conditions a therapeutically effective amount of a compound of the general formula I

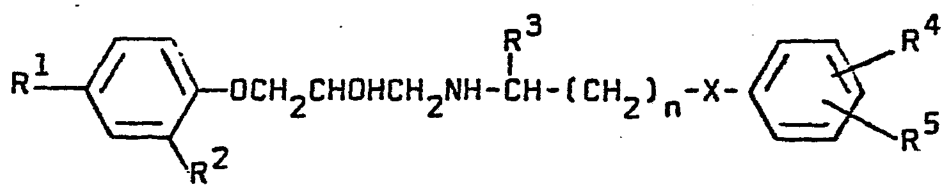

(I)

wherein $R^1$ is selected from the group consisting of alkoxyalkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl and hydroxy, n is an integer 0, 1, 2, or 3, and X is -O-, $-\overset{O}{\overset{\|}{C}}-$, or $-CH_2-$, whereby n is not zero when X is oxygen or its therapeutically acceptable acid addition salts.

11. A method according to clause 10, wherein
1) 3-[2-phenethylamino]-1-[4-(2-methoxyethyl)phenoxy]-propanol-2,
2) 3-[2-(4-hydroxyphenyl)-ethylamino]-1-[4-(2-methoxyethyl)-phenoxy]-propanol-2,
3) 3-[2-phenethylamino]-1-[4-(2-methoxyethyl)-2-bromophenoxy]-propanol-2,
4) 3-[1-methyl-2-(4-hydroxyphenyl)-ethylamino]-1-[4-(2-methoxyethyl)-2-bromophenoxy]-propanol-2,
5) 3-[1-methyl-2-(4-hydroxyphenyl)-ethylamino]-1-[4-(2-methoxyethyl)-2-chlorophenoxy]-propanol-2,

6) 3-[2-(4-hydroxyphenoxy)-ethylamino]-1-[4-(2-methoxyethyl)-phenoxy]-propanol-2,

7) 3-[2-(4-hydroxyphenoxy)-ethylamino]-1-[4-(2-methoxyethyl)--2-bromophenoxy]-propanol-2,

8) 3-[2-(4-hydroxyphenylcarbonyl)-ethylamino]-1-[4-(2--methoxyethyl)phenoxy]-propanol-2,

9 ) 3-[2-(3,5-dimethoxyphenyl)-ethylamino]-1-[4-(2-hydroxyethyl)phenoxy]-propanol-2,

10) 3-[2-(2-cyano-5-methylphenyl)-ethylamino]-1-(4-ethyl-phenoxy)-propanol-2,

11) 3-[2-(3,5-diethylphenylcarbonyl)-ethylamino]-1-(4-ethyl-2--bromophenoxy)-propanol-2,

12) 3-(2-phenethylamino)-1-[4-(2-methoxy-1-methylethyl)-phenoxy]-propanol-2,

13) 3-(2-phenethylamino)-1-[4-methoxymethyl)phenoxy]-propanol--2,

14) 3-(3-phenylpropylamino)-1-[4-(2-methoxyethyl)phenoxy]-propanol-2,

or its therapeutically acceptable salts is administered.

12. Pharmaceutical preparation which comprises as an active ingredient a therapeutically effective dose of at least one membrane stabilizing phenoxyhydroxypropylamine compound of the formula I

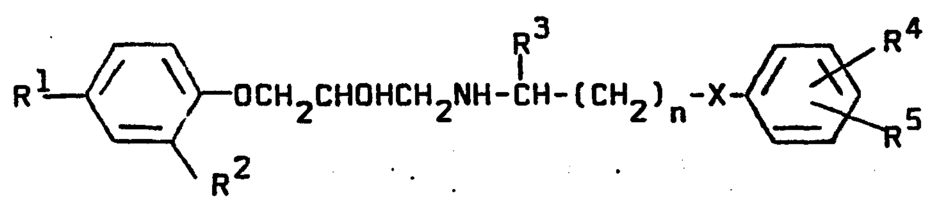

(I)

wherein $R^1$ is selected from the group consisting of alkoxy-alkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, hydroxy, alkoxy, alkoxyalkyl, cyano and alkyl, n is an integer 0, 1, 2, or 3, and X is -O-, $-\overset{\overset{O}{\|}}{C}-$, or $-CH_2-$, whereby n is not zero, when X is oxygen, in association with a pharmaceutically acceptable carrier.

13. A pharmaceutical preparation according to clause 12, wherein the active ingredient is a therapeutically effective dose of at least one of said compounds in racemic form.

14. A pharmaceutical preparation according to clause 12, wherein the active ingredient is a therapeutically effective dose of at least one of said compounds as the optically active, dextro-rotatory isomer.

15. A pharmaceutical preparation according to clause 12, wherein the active ingredient is a therapeutically effective dose of at least one of said compounds as the optically active, levo-rotatory isomer.

16. A pharmaceutical preparation according to clause 12, wherein the substituted phenoxy-hydroxypropylamine compound comprises 0,1 to 99% by weight of the preparation.

17. A pharmaceutical preparation according to clause 12 in a form suitable for administration by injection wherein the substituted phenoxy-hydroxypropylamine compound comprises about 0,5% to about 20% by weight of the preparation.

18. A pharmaceutical preparation according to clause 17 for parenteral application which comprises an aqueous solution of a water soluble salt of said substituted phenoxy-hydroxypropylamine compound in an amount of about 0,5-10% by weight of the preparation.

19. A pharmaceutical preparation according to clause 12 in a form suitable for oral administration wherein the substituted phenoxy-hydroxypropylamine compound comprises about 0,2% to

about 50% by weight of the preparation.

20. A pharmaceutical preparation according to clause 12, wherein the active ingredient is 3-(2-phenethylamino)-1-[4-(2-methoxy-1-methylethyl)phenoxy]-propanol-2 or a pharmaceutically acceptable non-toxic addition salt thereof.

21. A pharmaceutical preparation according to clause 12, wherein the active ingredient is 3-[2-phenethylamino]-1-[4-(2-methoxyethyl)phenoxy]-propanol-2 or a pharmaceutically acceptable non-toxic addition salt thereof.

22. A pharmaceutical preparation according to clause 12, wherein the active ingredient is 3-[2-(4-hydroxyphenyl)-ethylamino]-1-[4-(2-methoxyethyl)phenoxy]-propanol-2 or a pharmaceutically acceptable non-toxic addition salt thereof.

23. A pharmaceutical preparation according to clause 12, wherein the active ingredient is 3-[2-phenethylamino]-1-[4-(2-methoxyethyl)-2-bromophenoxy]-propanol-2 or a pharmaceutically acceptable non-toxic addition salt thereof.

24. A pharmaceutical preparation according to clause 12, wherein the active ingredient is 3-[1-methyl-2-(4-hydroxyphenyl)-ethylamino]-1-[4-(2-methoxyethyl)-2-bromophenoxy]-propanol-2 or a pharmaceutically acceptable non-toxic addition salt thereof.

25. A pharmaceutical preparation according to clause 12, wherein the active ingredient is 3-[1-methyl-2-(4-hydroxyphenyl)-ethylamino]-1-[4-(2-methoxyethyl)-2-chlorophenoxy]-propanol-2 or a pharmaceutically acceptable non-toxic addition salt thereof.

26. A pharmaceutical preparation according to clause 12, wherein the active ingredient is 3-[2-(4-hydroxyphenoxy)-ethylamino]-1-[4-(2-methoxyethyl)-phenoxy]-propanol-2 or a pharmaceutically acceptable non-toxic addition salt thereof.

27. A pharmaceutical preparation according to clause 12, wherein the active ingredient is 3-[2-(4-hydroxyphenoxy)ethylamino]-1-[4-(2-methoxyethyl)-2-bromophenoxy]-propanol-2 or a pharmaceutically acceptable non-toxic addition salt thereof.

28. A pharmaceutical preparation according to clause 12, wherein the active ingredient is 3-[2-(4-hydroxyphenylcarbonyl)-ethylamino]-1-[4-(2-methoxyethyl)phenoxy]-propanol-2 or a pharmaceutically acceptable non-toxic addition salt thereof.

29. A pharmaceutical preparation according to clause 12, wherein the active ingredient is 3-[2-(3,5-dimethoxyphenyl)-ethylamino]-1-[4-(2-hydroxyethyl)phenoxy]-propanol-2 or a pharmaceutically acceptable non-toxic addition salt thereof.

30. A pharmaceutical preparation according to clause 12, wherein the active ingredient is 3-[2-(2-cyano-5-methylphenyl)-ethylamino]-1-(4-ethylphenoxy)-propanol-2 or a pharmaceutically acceptable non-toxic addition salt thereof.

31. A pharmaceutical preparation according to clause 12, wherein the active ingredient is 3-[2-(3,5-diethylphenylcarbonyl)-ethylamino]-1-(4-ethyl-2-bromophenoxy)-propanol-2, or a pharmaceutically acceptable non-toxic addition salt thereof.

32. A pharmaceutical preparation according to clause 12, wherein the active ingredient is 3-(2-phenethylamino)-1-[4--(2-methoxy-1-methylethyl)phenoxy]-propanol-2 or a pharmaceutically acceptable non-toxic addition salt thereof.

33. A pharmaceutical preparation according to clause 12, wherein the active ingredient is 3-(2-phenethylamino)-1-[4--(methoxymethyl)phenoxy]-propanol-2, or pharmaceutically

acceptable non-toxic addition salt thereof.

34. A pharmaceutical preparation according to clause 12, wherein the active ingredient is 3-(3-phenylpropylamino)-1--[4-(2-methoxyethyl)phenoxy]-propanol-2, or a pharmaceutically acceptable non-toxic addition salt thereof.

35. A process for preparing new compounds according to clauses 1 to 2 and substantially as described.

36. Compounds according to clauses 3 to 9 and substantially as described.

37. A method of treating symptoms and signs of cardiac failure by stimulating the β-receptors of the heart and/or by blocking the β-receptors of the heart according to clauses 10 to 11 and substantially as described.

38. Pharmaceutical preparations according to clauses 12 to 35 and substantially as described.

## Claims

1. A process for the preparation of new amines of the formula

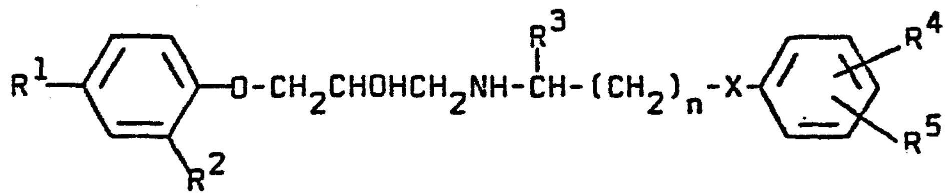

(I)

wherein $R^1$ is selected from the group consisting of alkoxyalkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl and hydroxy, n is an integer 0, 1, 2, or 3, and X is -O-, $\overset{O}{\overset{\|}{C}}$, or $-CH_2-$ whereby n is not zero when X is oxygen characterized in that

a) a compound of the formula II

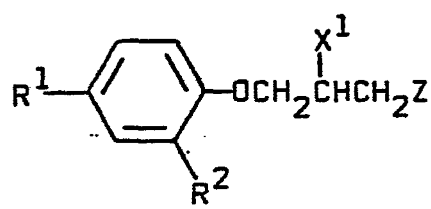

(II)

wherein $R^1$ and $R^2$ have the same meanings given above, $X^1$ is a hydroxy group and Z is a reactive, esterified hydroxy group, or $X^1$ and Z together form an epoxy group, is allowed to react with an amine of the formula

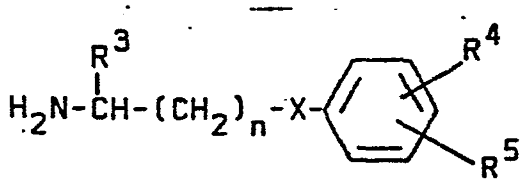

wherein $R^3$, $R^4$, $R^5$, n and X have the same meanings as given above, or

0007294

b) a compound of the formula III

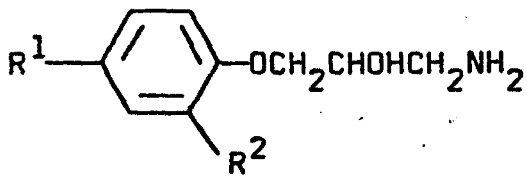

(III)

wherein $R^1$, and $R^2$ have the same meanings as given above, is allowed to react with a compound of the formula

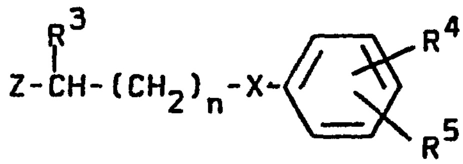

wherein $R^3$, $R^4$, $R^5$, n, X, and Z have the same meanings as given above,

c) a compound of the formula IV

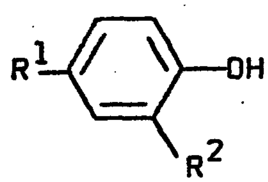

(IV)

wherein $R^1$, and $R^2$ have the same meanings as given above, is allowed to react with a compound of formula V

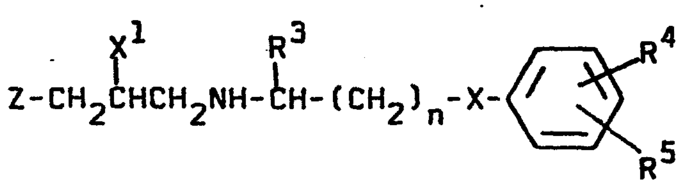

(V)

wherein Z, $X^1$, $R^3$, $R^4$, $R^5$, n, and X have the same meanings as given above, or

d) a compound of the formula IV

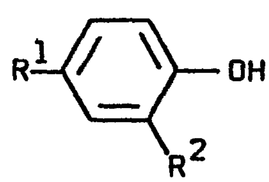

(IV)

wherein $R^1$, and $R^2$ have the same meanings as given above, is allowed to react with a compound of the formula VI

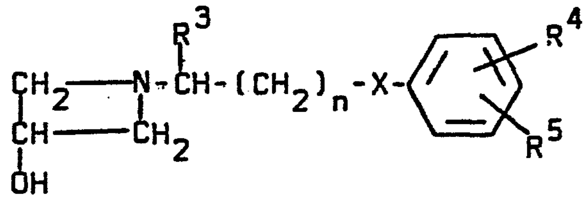

(VI)

wherein $R^3$, $R^4$, $R^5$, n and X have the same meanings as given above,

e) from a compound of the formula I wherein $R^1$, $R^2$, $R^3$, $R^4$, $R^5$, n, and X have the same meanings as above, and which compound has a removable residue on the nitrogen atom of the amino group and/or has a removable residue on the hydroxy groups, this residue is split off,

f) a Schiff's base of the formula VIII or IX

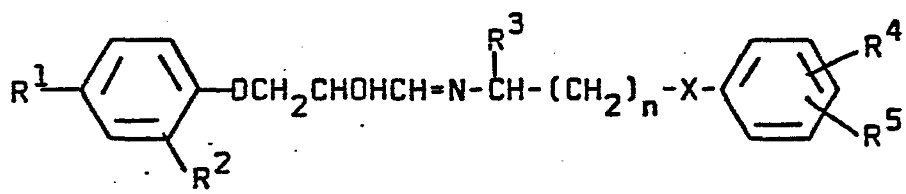

(VIII)

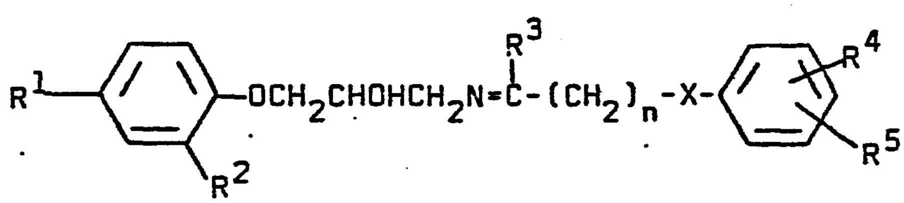

(IX)

or a cyclic tautomer X related to the compound of formula IX

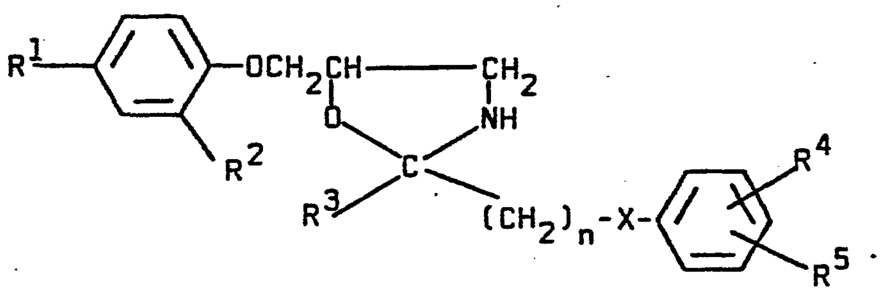

(X)

wherein $R^1$, $R^2$, $R^3$, $R^4$, $R^5$, n, and X have the same meanings

as above, except for the case when n = 0 and X = 0, whereby the compounds IX and X may be present simultaneously, is reduced, or

g) in a compound of the formula XI

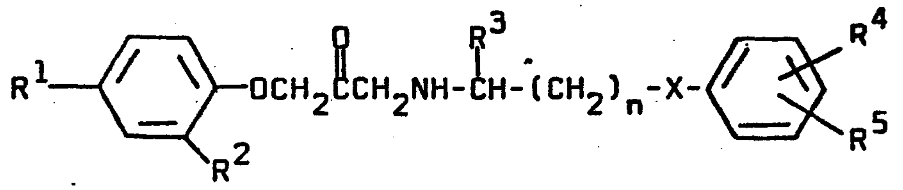

(XI)

wherein $R^1$, $R^2$, $R^3$, $R^4$, $R^5$, n, and X have the same meanings as above, the oxo group is reduced to a hydroxy group,

h) in a compound of the formula XII

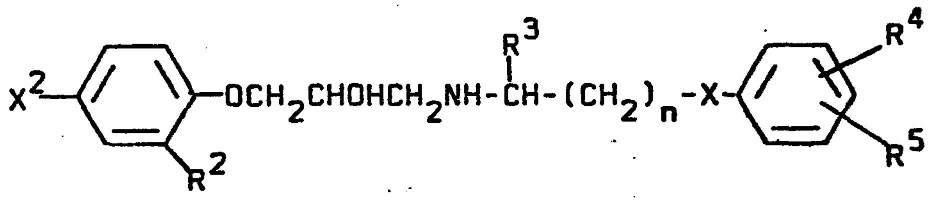

wherein $R^2$, $R^3$, $R^4$, $R^5$, n, and X have the same meanings as above and wherein $X^2$ is a residue transformable into $R^1$ having the above meanings, $X^2$ is transformed into $R^1$, or

i) a compound of formula Va

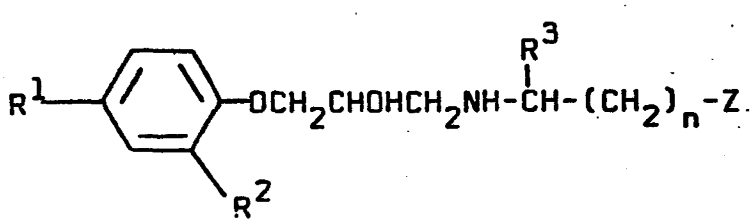

(Va)

wherein $R^1$, $R^2$, $R^3$, n, and Z have the same meanings as above is allowed to react with a compound of formula IVa

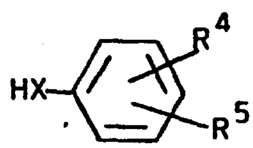

(IVa)

wherein $R^4$, and $R^5$ have the same meanings as above, and X is O; or

k) in a compound corresponding to the one of formula I having an oxo group at a C-atom adjacent to the N-atom, this oxo group is reduced to two hydrogen atoms, and

if desired, isomer mixtures obtained are separated into pure isomers, and/or racemates obtained are separated into optical antipodes, and/or free bases obtained are transformed into their therapeutically acceptable salt, or salts obtained are transformed into their free bases.

2. Compounds of the formula I

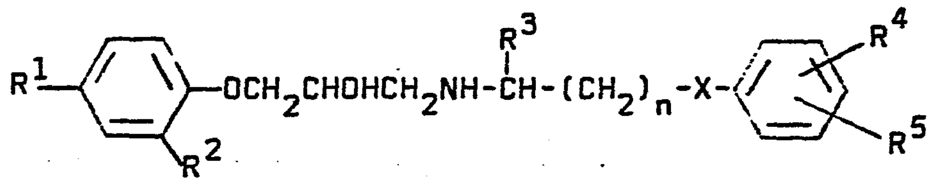 (I)

wherein $R^1$ is selected from the group consisting of alkoxyalkyl, hydroxyalkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl, and hydroxy, n is an integer 0, 1, 2, or 3, and X is -O-, $-\overset{O}{\overset{\|}{C}}-$, or $-CH_2-$, whereby n is not zero,when X is oxygen

3. A compound according to claim 2 in the form of a dextrorotating optical antipode.

4. A compound according to claim 2 in the form of a levorotating antipode.

5. A compound according to any one of claims 2-4 in the form of the free base.

6. A compound according to any one of claims 2-4 in the form of a salt.

7. A compound according to any one of claims 2-4 in the form of a therapeutically acceptable salt.

8. The method for treating symptoms and signs of arrhythmic conditions by stabilizing membranes of the heart and/or by blocking β-receptors of the heart by administering to mammals, including man, suffering from symptoms and signs of arrhythmic conditions a therapeutically effective amount of a compound of the general formula I

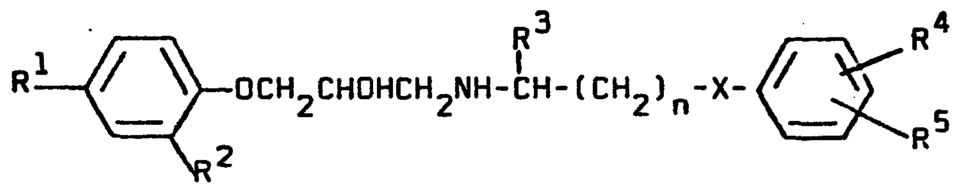
(I)

wherein $R^1$ is selected from the group consisting of alkoxy-alkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, alkoxy, alkoxyalkyl, cyano, alkyl and hydroxy, n is an integer 0, 1, 2, or 3, and X is -O-, $-\overset{O}{\overset{\|}{C}}-$, or $-CH_2-$, or its therapeutically acceptable acid addition salts.

9. Pharmaceutical preparation which comprises as an active ingredient a therapeutically effective dose of at least one membrane stabilizing phenoxyhydroxypropylamine compound of the formula I

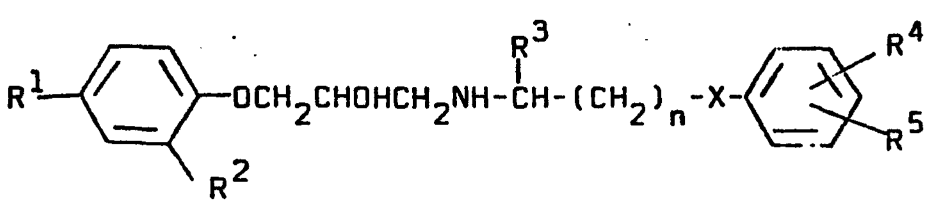
(I)

wherein $R^1$ is selected from the group consisting of alkoxyalkyl, hydroxyalkyl and alkyl, $R^2$ is selected from the group consisting of hydrogen and halogen, $R^3$ is selected from the group consisting of hydrogen and alkyl, $R^4$ and $R^5$ are the same or different and are each selected from the group consisting of hydrogen, hydroxy, alkoxy, alkoxyalkyl, cyano and alkyl, n is an integer 0, 1, 2, or 3, and X is -O, $-\overset{O}{\overset{\|}{C}}-$, or $-CH_2-$, in association with a pharmaceutically acceptable carrier.

10. A pharmaceutical preparation according to claim 12, wherein the substituted phenoxy-hydroxypropylamine compound comprises 0,1 to 99% by weight of the preparation.